# EUROPEAN PATENT APPLICATION

(11) **EP 4 488 248 A1**
(43) Date of publication of application: **08.01.2025**
(21) Application number: 23762135.4
(22) Date of filing: 28.02.2023
(51) Int. Cl.: C05F 1/00, C05G 5/20

(54) **FERTILIZER AND MANUFACTURING METHOD THEREOF**

(30) Priority: 01.03.2022 JP 2022031179
(71) Applicant: Kinki University, Higashi-Osaka-shi Osaka 577-8502 (JP)
(72) Inventor: MORIMOTO, Koichi, Kinokawa-shi, Wakayama 649-6493 (JP); SAKAMOTO, Masaru, Kinokawa-shi, Wakayama 649-6493 (JP); TAGUCHI, Yoshitomo, Kinokawa-shi, Wakayama 649-6493 (JP)
(74) Representative: Graf von Stosch Patentanwaltsgesellschaft mbH
(86) International application number: PCT/JP2023/007245
(87) International publication number: WO 2023/167166

(57) **Abstract**

Provided is a production method for producing a novel fertilizer (e.g., liquid fertilizer) containing a bone tissue degradation product. A production method for producing a fertilizer containing phosphoric acid in accordance with an aspect of the present invention includes at least one of: a step 1 (S7) of producing a fertilizer from a bone solubilization liquid A which has been obtained by treating bone tissue with a solution containing both an acid and a protease; a step 2 (S2) of producing a fertilizer from an acid extract which has been obtained by treating bone tissue with an acid; and a step 3 (S5) of producing a fertilizer from a protease treatment liquid which has been obtained by treating, with a protease, bone tissue which has been treated with an acid.

## Description

### Technical Field

The present invention relates to a fertilizer and a method for producing the fertilizer.

### Background Art

Conventionally, bone meal produced through high-temperature treatment or high-pressure treatment has been known as a fertilizer using bone tissue as a raw material (see, e.g., Patent Literature 1).

### Citation List

### [Patent Literature]

[Patent Literature 1]
Japanese Patent Application Publication Tokukai No. 2005-247616

### Summary of Invention

### Technical Problem

Bone tissue contains, in addition to phosphoric acid, an abundance of inorganic components (such as calcium, potassium, sodium, and vitamin) and organic components (such as protein, proteoglycan, fatty acid, polysaccharide, and monosaccharide). In addition to the above mentioned substances, bone tissue also contains: bone-related cells (such as bone cells, osteoblasts, and osteoclasts); immunocytes (such as T cells, macrophages, and leukocytes); debris of red blood cells, adipocytes, stem cells, and the like; bone marrow and blood components remaining in bone tissue; and the like. As such, bone tissue is an aggregate of various inorganic components and organic components. That is, a bone tissue degradation product contains, in addition to phosphoric acid, a plurality of molecules which are effective for plant growth, and it is thus highly possible that a synergistic effect would be brought about. Therefore, a fertilizer containing a bone tissue degradation product can be a high-value added product. Conventionally, fertilizers are known such as degradation products of food residues by microorganisms and leaf mold by compost. However, a contained amount of inorganic components is relatively small in food residues, and a resultant fertilizer also tends to contain a small amount of inorganic components. Moreover, degradation of food residues by microorganisms takes a long time, and generation of an odor is also a major problem. Furthermore, existing bone-derived fertilizers are slow-acting and often used in soil improvement over several years. Under the circumstances, it has been demanded to develop a fertilizer which is fast-acting, simple, and inexpensive, and promotes plant growth.

However, a production method in which high-temperature treatment or high-pressure treatment is carried out as in the technique disclosed in Patent Literature 1 involves a high operation cost, and it is difficult to control production. Furthermore, geographical conditions for a manufacturing plant are limited. Under the circumstances, the inventors of the present invention have conceived of degrading bone tissue without involving high-temperature treatment or high-pressure treatment, and using a resultant bone tissue degradation product as a fertilizer. Herein, fertilizers refer to both a solid fertilizer and a liquid fertilizer. The bone tissue degradation product may be used as a liquid fertilizer which is a diluted solution, or may be used as a solid fertilizer obtained through a step such as lyophilization or air drying. A product which has been obtained by spraying a bone tissue degradation product onto an existing solid fertilizer or immersing an existing solid fertilizer in a bone tissue degradation product may be used as a fertilizer.

Existing fertilizers using bone tissue as a raw material, including bone meal which has been treated with high temperature or high pressure, are solid fertilizers. A solid fertilizer is used while being mixed in culture soil, and is therefore not suitable for hydroponic culture. If a solid fertilizer is used in hydroponic culture, a dissolution rate of the fertilizer varies. Therefore, there is a possibility that an imbalance in concentration of the fertilizer would occur, and a place which includes an excessive amount of fertilizer and a place which is short of fertilizer would occur. Furthermore, a solid fertilizer cannot be used in a fertilization form such as foliar spray, and a plant growth effect thereof is slow-acting. Thus, there are several difficulties in existing solid fertilizers using bone tissue as a raw material.

An object of an aspect of the present invention is to provide a novel fertilizer (e.g., liquid fertilizer) containing a bone tissue degradation product.

### Solution to Problem

< 1> A production method for producing a fertilizer containing phosphoric acid, the production method including at least one of:
   a step 1 of producing a fertilizer from a bone solubilization liquid A which has been obtained by treating bone tissue with a solution containing both an acid and a protease;
   a step 2 of producing a fertilizer from an acid extract which has been obtained by treating bone tissue with an acid; and
   a step 3 of producing a fertilizer from a protease treatment liquid which has been obtained by treating, with a protease, bone tissue which has been treated with an acid.
<2> The production method described in <1>, further including, in addition to the step 2 and the step 3:
   a step 4 of producing a fertilizer from a bone solubilization liquid B which has been obtained by mixing the acid extract and the protease treatment liquid.
<3> The production method described in <1> or <2>, in which: the production method includes the step 1 or the step 2; and bone tissue is treated with an acid which is at least one selected from the group consisting of nitric acid, hydrochloric acid, formic acid, and sulfuric acid.
<4> The production method described in any one of < 1 > through <3>, in which: the production method includes the step 1 or the step 3; and bone tissue is treated with a protease having an optimum pH of 1.5 to 8.0.
<5> The production method described in any one of <1> through <4>, in which: the production method includes the step 1 or the step 2; and bone tissue is treated with an acid at 5°C to 60°C.
<6> The production method described in any one of < 1 > through <5>, in which: the production method includes the step 1 or the step 2; and bone tissue is treated with an acid of 0.6 mol/L to 2.0 mol/L.
<7> The production method described in any one of <1> through <6>, in which: the production method includes the step 1 or the step 2; and bone tissue is treated with an acid for 6 hours to 48 hours.
<8> The production method described in any one of < 1 > through <7>, further including: a pretreatment step of pretreating the bone tissue prior to the step 1 or the step 2, the pretreatment being at least one selected from the group consisting of heating of the bone tissue, heating of the bone tissue under pressure, and irradiation of the bone tissue with microwaves.
<9> The production method described in any one of < 1 > through <8>, in which: in the pretreatment step, a protein contained in the bone tissue is denatured.
<10> A fertilizer which is obtained by the production method described in any one of <1> through <9>.
<11> The fertilizer described in <10>, in which: the fertilizer is a liquid fertilizer.
<12> A fertilizer, containing: phosphoric acid; and a peptide fragment which is derived from at least one selected from the group consisting of type I collagen, alpha-2-HS-glycoprotein, periostin, biglycan, and SPARC.
<13> The fertilizer described in < 12 >, in which: in the peptide fragment, activity of a protein from which the peptide fragment is derived is lost.
< 14> The fertilizer described in <12> or < 13 >, in which: a molecular weight of the peptide fragment is 10,000 Da or less.
<15> The fertilizer described in any one of < 12 > through <14>, in which: a concentration of the phosphoric acid contained in the fertilizer is 280 mM or more.

### Advantageous Effects of Invention

According to an aspect of the present invention, it is possible to provide a novel fertilizer (e.g., liquid fertilizer) containing a bone tissue degradation product.

### Brief Description of Drawings

Fig. 1 is a flowchart illustrating an example of a production method for producing a fertilizer in accordance with an embodiment of the present invention.
Fig. 2 is a flowchart illustrating another example of a production method for producing a fertilizer in accordance with an embodiment of the present invention.
Fig. 3 is a flowchart illustrating an example of a production method for producing phosphoric acid in accordance with an embodiment of the present invention.
Fig. 4 is a diagram illustrating a result of treating, with a protease, bone tissue which has been obtained by treating a pig bone with nitric acid.
Fig. 5 is a diagram illustrating a result of treating, with actinidain, bone tissue which has been obtained by treating a pig bone with nitric acid or hydrochloric acid.
Fig. 6 is a diagram illustrating a result of preparing, using various kinds of acids and proteases, bone solubilization liquids A without involving solution replacement.
Fig. 7 is a diagram illustrating a result of an experiment for studying a plant growth effect by an acid extract.
Fig. 8 is a diagram illustrating a result of an experiment for studying a plant growth effect by an acid extract, a protease treatment liquid, or a bone solubilization liquid A.
Fig. 9 is a diagram illustrating a result of an experiment for comparing plant growth effects by a bone solubilization liquid A and a commercially available culture solution.

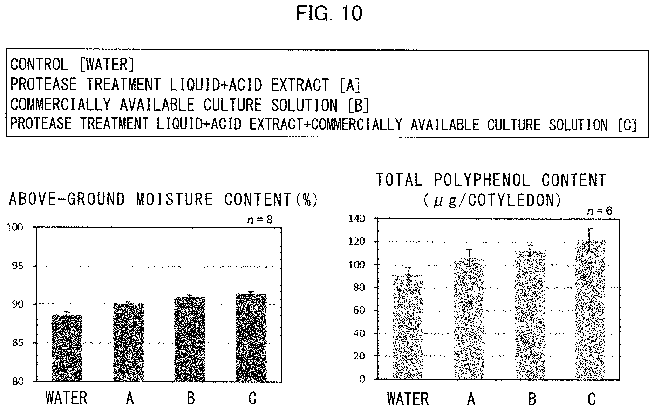

Fig. 10 is a diagram illustrating a result of an experiment for comparing plant growth effects by a bone solubilization liquid A and a commercially available culture solution.
Fig. 11 is a diagram illustrating a result of an experiment for comparing changes in expression amount of a stress tolerance-related gene by a bone solubilization liquid A and a commercially available liquid fertilizer.
Fig. 12 is a diagram illustrating a result of an experiment for comparing changes in expression amount of a stress tolerance-related gene by a bone solubilization liquid A and a commercially available liquid fertilizer.
Fig. 13 is a diagram illustrating influence of pretreatment of bone tissue on an amount of phosphoric acid contained in an acid extract.
Fig. 14 is a diagram illustrating a result of analyzing, by electrophoresis, a protein component contained in an acid extract.
Fig. 15 is a diagram illustrating a result of preparing protease treatment liquids using various kinds of proteases.
Fig. 16 is a diagram illustrating a result of analyzing, by electrophoresis, a protein component contained in a protease treatment liquid.

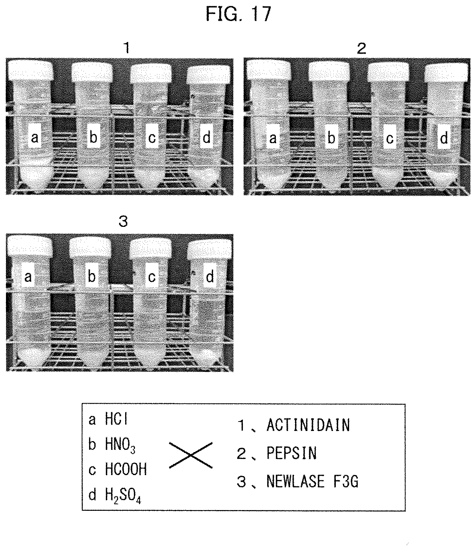

Fig. 17 is a diagram illustrating a result of preparing, using various kinds of acids and proteases, bone solubilization liquids A without involving solution replacement.
Fig. 18 is a diagram illustrating a result of analyzing an expression variable gene in a leaf of a sprout which was cultivated for 5 days while providing a bone solubilization liquid B or a commercially available liquid fertilizer.
Fig. 19 is a diagram illustrating a result of analyzing an expression variable gene in a root of a sprout which was cultivated for 5 days while providing a mixed solution of a bone solubilization liquid B and a commercially available liquid fertilizer or only a commercially available liquid fertilizer.
Fig. 20 is a diagram illustrating a result of analyzing an expression variable gene in a leaf of a sprout which was cultivated for 5 days while providing a mixed solution of a bone solubilization liquid B and a commercially available liquid fertilizer or only a commercially available liquid fertilizer.
Fig. 21 is an elution curve indicating a result of extracting phosphoric acid while using commercially available bone meal as a raw material.
Fig. 22 is a diagram illustrating a result of producing a liquid fertilizer while using commercially available bone meal as a raw material. As a protease, Newlase F3G was used.
Fig. 23 is a diagram illustrating a result of producing a liquid fertilizer while using commercially available bone meal as a raw material. As a protease, papain was used.

### Description of Embodiments

### [1. Production method for producing fertilizer]

A production method for producing a fertilizer in accordance with an aspect of the present invention includes at least one of steps 1 through 3 below. In an embodiment, the production method for producing a fertilizer further includes a step 4 below. The following description will sequentially discuss details of the step 2, the step 3, the step 4, and the step 1 in this order.

Step 1 of producing a fertilizer from a bone solubilization liquid A which has been obtained by treating bone tissue with a solution containing both an acid and a protease.

Step 2 of producing a fertilizer from an acid extract which has been obtained by treating bone tissue with an acid.

Step 3 of producing a fertilizer from a protease treatment liquid which has been obtained by treating, with a protease, bone tissue which has been treated with an acid.

Step 4 of producing a fertilizer from a bone solubilization liquid B which has been obtained by mixing the acid extract and the protease treatment liquid.

### [1.1. Step 2]

The step 2 is a step of producing a fertilizer from an acid extract which has been obtained by treating bone tissue with an acid. In the step 2, an inorganic component is mainly separated from bone tissue, and eluted into an acid extract. From the obtained acid extract, a fertilizer is produced. The acid extract itself may be used as a fertilizer, or a bone solubilization liquid B in which the acid extract is mixed with a protease treatment liquid may be used as a fertilizer.

Bone tissue subjected to the step 2 can be derived from any organism. Examples of the organism include mammals, birds, amphibians, and fish. In order to produce a fertilizer in a large amount, it is preferable to obtain a large amount of bone tissue which is used as a raw material of a fertilizer. For example, bone tissue of a domestic animal (cattle, pig, sheep, chicken, and the like) is suitably used. Prior to treating bone tissue with an acid, the bone tissue may be shredded or pulverized in advance. With such pretreatment, bone tissue degradation can be carried out more efficiently. Therefore, it may be possible to reduce a production time.

An acid for treating bone tissue in the step 2 is not particularly limited. Examples of the acid include hydrochloric acid, nitric acid, formic acid, sulfuric acid, and trichloroacetic acid. An acidic decalcifying solution such as a Plank-Rychlo solution may be used. In view of availability, it is preferable to use at least one selected from the group consisting of nitric acid, hydrochloric acid, formic acid, and sulfuric acid. In view of the recovery efficiency of phosphoric acid, at least one selected from the group consisting of hydrochloric acid, sulfuric acid, and nitric acid is preferable, and at least one selected from the group consisting of hydrochloric acid and nitric acid is more preferable. In view of recovery efficiency of calcium, it is preferable to use at least one selected from the group consisting of hydrochloric acid and formic acid. Two or more types of acids may be mixed and used in an appropriate ratio.

In the step 2, bone tissue may be treated with an acid by a solution in which an acid is diluted in a solvent. Examples of the solvent include water, lower alcohol, glycerol, propane-1,2-diol, and 1,3-propanediol. Two or more types of solutions may be mixed and used in an appropriate ratio.

An acid concentration can be determined as appropriate according to a volume of bone tissue which is to be treated with an acid. In a case where the volume of bone tissue is small, treatment can be carried out with an acid of low concentration. In a case where the volume of bone tissue is large, it is preferable to use an acid of high concentration. In a case where acid treatment is carried out with an acid of low concentration, it is preferable to treat bone tissue in the form of fine powder. However, even in a case where the volume of bone tissue is large, an inorganic component can be sufficiently extracted by heightening the acid concentration and immersing the bone tissue for a long time. For example, in a case where bone fragments having a volume of several cubic centimeters or less are used as a raw material, a lower limit of acid concentration in the step 2 can be 0.6 mol/L or more, 0.7 mol/L or more, 0.8 mol/L or more, or 0.9 mol/L or more. An upper limit of acid concentration in the step 2 can be 2.0 mol/L or less, 1.5 mol/L or less, 1.0 mol/L or less, or 0.9 mol/L or less. In a case where the acid concentration is in the above range, an inorganic component can be extracted efficiently, and a cost of neutralizing an acid extract can be reduced because the acid concentration is not too high.

Examples of a suitable acid concentration in a case where bone tissue in the form of fine powder is treated with an acid are as follows. In a case where the acid is nitric acid, a lower limit of nitric acid concentration is preferably 0.6 mol/L or more, more preferably 0.7 mol/L or more. An upper limit of nitric acid concentration is preferably 1.0 mol/L or less, more preferably 0.9 mol/L or less. In a case where the acid is hydrochloric acid, a lower limit of hydrochloric acid concentration is preferably 0.8 mol/L or more, more preferably 0.9 mol/L or more. An upper limit of hydrochloric acid concentration is preferably 1.2 mol/L or less, more preferably 1.1 mol/L or less. In a case where the acid is formic acid, a lower limit of formic acid concentration is preferably 0.8 mol/L or more, more preferably 0.9 mol/L or more. An upper limit of formic acid concentration is preferably 1.2 mol/L or less, more preferably 1.1 mol/L or less. In a case where the acid is sulfuric acid, a lower limit of sulfuric acid concentration is preferably 0.8 mol/L or more, more preferably 0.9 mol/L or more. An upper limit of sulfuric acid concentration is preferably 1.2 mol/L or less, more preferably 1.1 mol/L or less. The above concentrations are examples of a suitable concentration in a case where bone tissue is in the form of fine powder. In a case where bone tissue having a greater volume is used as a raw material, the acid concentration may be further heightened.

For example, in a case where bone fragments having a volume of several cubic centimeters or less are used as a raw material, a lower limit of extraction time in the step 2 is preferably 6 hours or more, more preferably 8 hours or more, further preferably 10 hours or more. An upper limit of extraction time in the step 2 is preferably 48 hours or less, more preferably 24 hours or less, further preferably 14 hours or less. In a case where the extraction time is in the above range, it is possible to sufficiently extract an inorganic component contained in bone tissue.

For example, in a case where bone fragments having a volume of several cubic centimeters or less are used as a raw material, a temperature of acid treatment in the step 2 is preferably 5°C to 60°C. In a case where acid treatment is carried out in the above temperature range, generation of an unpleasant odor associated with acid treatment can be reduced. Therefore, the geographical conditions for a fertilizer production factory are eased. In order to adjust the temperature conditions to be 60°C or less, an expensive special apparatus is not necessary, and the temperature can be kept constant by using a water bath or an incubator.

In the step 2, in addition to the acid, a chelating agent capable of trapping calcium ions may be added. Alternatively, in the step 2, bone tissue may be treated with a chelating agent before or after acid treatment (between the acid treatment and the chelating agent treatment, a solution may be or may not be replaced). Examples of the chelating agent include ethylenediaminetetraacetic acid (EDTA, CAS registration number: 60-00-4), glycol ether diaminetetraacetic acid (EGTA, CAS registration number: 67-42-5), and ethylenediamine-N,N'-disuccinic acid (EDDS, CAS registration number: 20846-91-7). In the step 2, a pH of the solution containing the chelating agent is preferably 6.0 to 8.0. For example, in a case where bone fragments having a volume of several cubic centimeters or less are used as a raw material, a lower limit of the concentration of the chelating agent in the step 2 can be 0.1 mol/L or more, 0.2 mol/L or more, 0.3 mol/L or more, or 0.4 mol/L or more. An upper limit of the concentration of the chelating agent in the step 2 can be 0.9 mol/L or less, 0.8 mol/L or less, 0.7 mol/L or less, or 0.6 mol/L or less.

In the step 2, at least a part of calcium may be removed after acid treatment. Examples of a method for removing calcium include: a method in which an acid extract is neutralized (a calcium phosphate precipitate is generated); a method in which sulfuric acid or sulfate is added (calcium sulfate precipitates); a method in which carbonic acid or carbonate is added (calcium carbonate or calcium hydrogen carbonate precipitates); and a method in which hydrogen carbonate is added (calcium carbonate or calcium hydrogen carbonate precipitates).

In a case where a pretreatment step (described later) is carried out, a phosphoric acid concentration of the acid extract obtained in the step 2 tends to improve. A concentration of phosphoric acid contained in the acid extract can be, for example, 280 mM or more, 300 mM or more, or 320 mM or more.

### [1.2. Step 3]

The step 3 is a step of producing a fertilizer from a protease treatment liquid which has been obtained by treating, with a protease, bone tissue which has been treated with an acid. From the obtained protease treatment liquid, a fertilizer is produced. The protease treatment liquid itself may be used as a fertilizer, or a bone solubilization liquid B in which the protease treatment liquid is mixed with an acid extract may be used as a fertilizer.

A protease used in the step 3 is not particularly limited. Examples of the protease include serine protease, cysteine protease, aspartic protease, glutamic protease, and metalloprotease.

Specific examples of the protease include trypsin [EC 3.4.21.4], chymotrypsin [EC 3.4.21.1], [EC 3.4.21.2], pepsin [EC 3.4.23.1], ecolicin [EC 3.4.23.19], papain [EC 3.4.22.2], ficin [EC 3.4.22.3], actinidain [EC 3.4.22.14], bromelain [EC 3.4.22.32], cathepsin B [EC 3.4.22.1], cathepsin H [EC 3.4.22.16], cathepsin K [EC 3.4.22.38], cathepsin L [EC 3.4.22.15], cathepsin S [EC 3.4.22.27], and thermolysin [EC 3.4.24.27].

As the protease, it is possible to use a commercially available enzyme formulation. Examples of such a formulation include Newlase F3G (derived from Rhizopus niveus), Orientase AY (derived from Aspergillus niger), Tetrase (derived from Aspergillus niger), Sumizyme AP (derived from Aspergillus niger), Denapsin 2P (derived from Aspergillus), Brewers Clarex (derived from Aspergillus niger), Maxipro AFP (derived from Aspergillus niger), Protease S "Amano" G (derived from Bacillus stearothermophilus), Protease N "Amano" G (derived from Bacillus subtilis), Protease NL "Amano" (derived from Bacillus subtilis), Protease A "Amano" G (derived from Aspergillus oryzae), Umamizyme (derived from Aspergillus oryzae), Protease P "Amano" 3G (derived from Aspergillus melleus), Protease M "Amano" SD (derived from Aspergillus oryzae), and Peptidase R (derived from Rhizopus oryzae). Alternatively, an enzyme which has been chemically synthesized, an enzyme which has been produced by a technique of genetic engineering, or an enzyme extracted from an organism may be used.

The protease used in the step 3 is preferably a protease having an optimum pH of 1.5 to 8.0. Examples of the protease having an optimum pH of 1.5 to 8.0 include Protease S "Amano" G (optimum pH: 7.0 to 8.5), Protease N "Amano" G (optimum pH: 6.0 to 7.5), Protease NL "Amano" (optimum pH: 6.5 to 7.5), Protease A "Amano" G (optimum pH: 6.0 to 7.5), Umamizyme (optimum pH: 6.0 to 7.5), Protease M "Amano" G (optimum pH: 3.0 to 6.5), Protease P "Amano" 3G (optimum pH: 7.0 to 8.0), Peptidase R "Amano" (optimum pH: 6.0 to 8.0), actinidain (optimum pH: 2.5 to 7.5), papain (optimum pH: 4.0 to 9.0), pepsin (optimum pH: 1.5 to 3.0), Newlase F3G (optimum pH: 3.0 to 5.0), trypsin (optimum pH: 7.0 to 9.0), and chymotrypsin (optimum pH: 7.0 to 9.0). The protease used is, more preferably, a protease having an optimum pH of 1.5 to 5.0. Examples of the protease having an optimum pH of 1.5 to 5.0 include Protease M "Amano" (optimum pH: 3.0 to 6.5), actinidain (optimum pH: 2.5 to 7.5), papain (optimum pH: 4.0 to 9.0), pepsin (optimum pH: 1.5 to 3.0), and Newlase F3G (optimum pH: 3.0 to 5.0). The protease used is, further preferably, a protease having an optimum pH of 1.5 to 4.0. Examples of the protease having an optimum pH of 1.5 to 4.0 include actinidain (optimum pH: 2.5 to 7.5), pepsin (optimum pH: 1.5 to 3.0), and Newlase F3G (optimum pH: 3.0 to 5.0).

A concentration of the protease in the step 3 can be set as appropriate. A lower limit of the protease concentration can be 2 mg/L or more or 10 mg/L or more. An upper limit of the protease concentration can be 100 mg/L or less or 50 mg/L or less.

A temperature and a pH in the step 3 can be set as appropriate. It is preferable to set the temperature and the pH to be an optimum temperature and an optimum pH of a protease used, in order to improve treatment efficiency. Examples of the temperature of the reaction system in the step 3 can be 20°C to 60°C. A lower limit of the temperature of the reaction system in the step 2 can be more than 10°C, 20°C or more, 25°C or more, 30°C or more, 35°C or more, 40°C or more, 45°C or more, 50°C or more, or 55°C or more. An upper limit of the temperature of the reaction system in the step 2 can be 60°C or less or 55°C or less.

In the step 3, a salt may be added to the reaction system. In accordance with a concentration of a salt added to the reaction system, substrate specificity of a protease may vary. Therefore, by adding a salt to the reaction system, it is possible to change a component contained in a resultant protease treatment liquid.

Examples of the salt added to the reaction system in the step 3 include a chloride salt. Examples of the chloride salt include NaCl, KCl, LiCl, and MgCl₂. A lower limit of a concentration of the salt added to the reaction system in the step 3 can be more than 0 mmol/L, 20 mmol/L or more, 100 mmol/L or more, 150 mmol/L or more, 200 mmol/L or more, 500 mmol/L or more, 1000 mmol/L or more, 1500 mmol/L or more, or 2000 mmol/L or more. An upper limit of the concentration of the salt added to the reaction system in the step 3 can be 4000 mmol/L or less or 2000 mmol/L or less.

In an embodiment, a size of a peptide fragment generated in the step 3 is 10,000 Da or less, 8,000 Da or less, 6,000 Da or less, or 4,000 Da or less. A peptide fragment which has been cleaved to such a size has highly probably lost physiological activity. Moreover, a peptide fragment which has been cleaved to such a size is easily absorbed by a plant as a nutrient, and may function as an active ingredient of a fertilizer. In an embodiment, the peptide fragment is derived from at least one selected from the group consisting of type I collagen, alpha-2-HS-glycoprotein, periostin, biglycan, and SPARC.

### [1.3. Step 4]

The step 4 is a step of producing a fertilizer from a bone solubilization liquid B which has been obtained by mixing the acid extract obtained in the step 2 with the protease treatment liquid obtained in the step 3. From the obtained bone solubilization liquid B, a fertilizer is produced. In the step 4, a mixing ratio of the acid extract and the protease treatment liquid is not particularly limited. A mixing ratio can be selected as appropriate so that a resultant bone solubilization liquid B has an intended composition.

### [1.4. Step 1]

The step 1 is a step of producing a fertilizer from a bone solubilization liquid A which has been obtained by treating bone tissue with a solution containing both an acid and a protease. In the step 1, acid treatment in the step 2 and protease treatment in the step 3 proceed simultaneously. In the step 1, it is not necessary to replace a solution between the acid treatment and the protease treatment.

In the step 1, an order in which bone tissue is brought into contact with an acid and a protease is not particularly limited. Examples of the order include orders below. Among these orders, order 1 is preferable.
Order 1: Bone tissue is immersed in a solution containing an acid, and after a predetermined time period has elapsed, a protease is added to the solution. Before adding the protease, a pH of the solution may be adjusted to an optimum pH of the protease.
Order 2: Bone tissue is immersed in a solution containing a protease, and after a predetermined time period has elapsed, an acid is added to the solution.
Order 3: A solution containing both an acid and a protease is prepared, and bone tissue is immersed in the solution.

For descriptions of preferable conditions of the acid treatment and the protease treatment in the step 1, the descriptions in sections [1.1] and [1.2] above can be applied.

### [1.5. Other step]

The production method for producing a fertilizer in accordance with an embodiment of the present invention may further include, in addition to steps 1 through 4, a step that can be usually carried out in producing a fertilizer. Examples of such a step include a pretreatment step, a component addition step, a drying step, a pulverization step, a coating-granulating step, and a packaging step.

The pretreatment step is a step preceding the step 1 or the step 2. In the pretreatment step, bone tissue which is a raw material is pretreated. An amount of phosphoric acid extracted from bone tissue can be increased by carrying out the pretreatment step (see Example 4).

In an embodiment, in the pretreatment step, bone tissue is heated. A heating temperature at this time can be 30°C or more or 40°C or more; 100°C or less or 80°C or less. Heating may be carried out in a state where bone tissue is immersed in an acid.

In an embodiment, in the pretreatment step, bone tissue is heated under pressure. A pressure at this time can be 200 kPa or more or 1 MPa or more; 500 MPa or less or 800 MPa or less. A heating temperature at this time can be 10°C or more or 50°C or more; 120°C or less or 200°C or less.

In an embodiment, in the pretreatment step, bone tissue is irradiated with microwaves. Heating, heating under pressure, and microwave irradiation may be carried out in an arbitrary combination. Among those, microwave irradiation is more preferable as a pretreatment step because an effect is brought about in a short time. A microwave irradiation time can be 5 seconds or more, 10 seconds or more, or 15 seconds or more; 10 minutes or less, 7 minutes or less, or 5 minutes or less.

In a case where the pretreatment step is carried out, a protein contained in bone tissue is denatured. Therefore, a fertilizer which is obtained by carrying out the pretreatment step contains a denatured protein (or a fragment thereof). The denatured protein (or a fragment thereof) has lost physiological activity which an original protein has.

The component addition step is a step of adding an additional fertilizer component. In a case where the component addition step is provided, it is possible to produce a fertilizer having a suitable composition in accordance with a plant to be cultivated. Examples of the fertilizer component added in the component addition step include potassium components (such as potassium oxide, potassium hydroxide, potassium chloride, and potassium sulfate), nitrogen components (such as urea and ammonium nitrate), magnesium components (such as magnesium phosphate, magnesium chloride, and magnesium sulfate), vitamins, manganese, boron, iron, copper, zinc, and molybdenum. The acid extract, the protease treatment liquid, the bone solubilization liquid A, or the bone solubilization liquid B may be mixed with another fertilizer (such as an inorganic fertilizer or an organic fertilizer).

The drying step is a step of removing excess water from the acid extract, the protease treatment liquid, the bone solubilization liquid A, or the bone solubilization liquid B. By carrying out the drying step, a fertilizer in the form of solid or paste is obtained. A solid fertilizer may be cut and pulverized, if necessary, to a size and a shape which promote fertilization.

The coating-granulating step is a step of coating and granulating a solid fertilizer. For example, in a case where a fertilizer is coated with a silicic acid compound or the like, it is possible to adjust a fertilizer effect time, prevent immobilization of phosphorus and calcium, prevent runoff of a fertilizer, and prevent damage to a fertilizer by impact.

The packaging step is a step of packaging the acid extract, the protease treatment liquid, the bone solubilization liquid A, or the bone solubilization liquid B in a container so that the liquid can be distributed or sold as a fertilizer. In the packaging step, the acid extract, the protease treatment liquid, the bone solubilization liquid A, or the bone solubilization liquid B may be combined with a manual for using the liquid as a fertilizer. This manual may be printed on the container or may be prepared as a physical or electronic document separately from a packaged fertilizer. In the manual, a formulation of a fertilizer, a fertilization method, a fertilization time, a target crop, and the like can be described.

### [1.6. Production method for producing an example fertilizer]

An example of a production method for producing a fertilizer in accordance with an embodiment of the present invention will be described in accordance with an example flowchart.

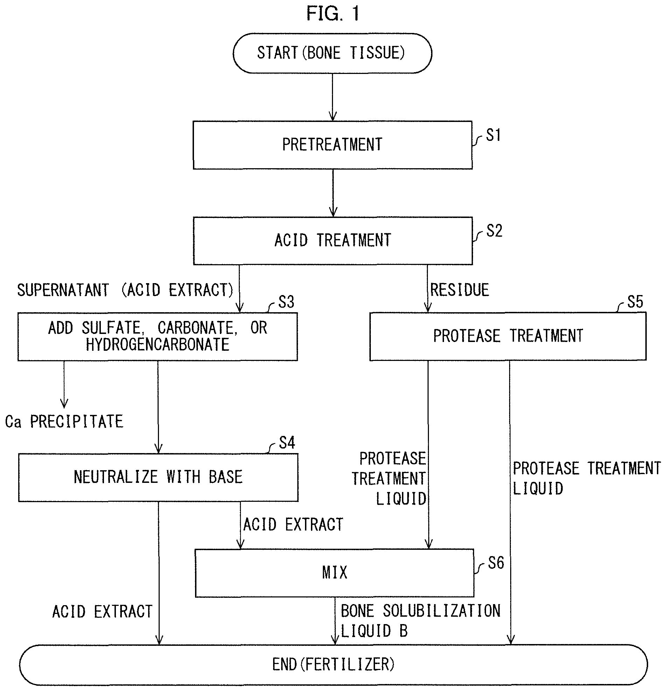

Fig. 1 is an example flowchart illustrating a production method including a step 2, a step 3, and/or a step 4. In this flowchart, an acid extract is obtained by carrying out step S1, step S2, step S3, and step S4. Moreover, by carrying out step S1, step S2, and step S5, a protease treatment liquid is obtained. Furthermore, by subjecting the acid extract and the protease treatment liquid to step S6, a bone solubilization liquid B is obtained. The acid extract, the protease treatment liquid, and the bone solubilization liquid B can all be used as components of a fertilizer or a mixed fertilizer. The acid extract contains phosphoric acid, which is an essential nutrient of a plant, and therefore the acid extract itself can be used as a fertilizer.

In step S1, bone tissue is pretreated. Step S1 is an optional step, and does not need to be carried out. An amount of phosphoric acid contained in an acid extract can be increased by pretreating bone tissue. This step is as described in the section [1.5] above.

In step S2, bone tissue is treated with an acid. A supernatant obtained through step S2 is an acid extract. Production of a fertilizer from the acid extract corresponds to the foregoing step 2. Treatment of bone tissue with an acid is as described in the section [1.1] above.

In step S3, sulfate, carbonate, or hydrogen carbonate is added. Step S3 is an optional step, and does not need to be carried out. By step S3, calcium ions contained in the acid extract precipitate as calcium sulfate, calcium carbonate, or calcium hydrogen carbonate. Examples of sulfate include sodium sulfate, potassium sulfate, ammonium sulfate, and magnesium sulfate. Examples of the carbonate include potassium carbonate and ammonium carbonate. Examples of the hydrogen carbonate include potassium hydrogen carbonate. Preferably, the sulfate is at least one selected from the group consisting of potassium sulfate, ammonium sulfate, and magnesium sulfate. Preferably, the carbonate is potassium carbonate. Preferably, the hydrogen carbonate is potassium hydrogen carbonate. In a case where these types of sulfate, carbonate, or hydrogen carbonate are used, potassium, magnesium, and/or ammonium are contained in the acid extract. These components are important nutrients for plants.

In step S4, the acid extract is neutralized with a base. Step S4 is an optional step, and does not need to be carried out. By step S4, the liquid nature of the acid extract is returned from acidic to substantially neutral. Examples of a base which can be used include sodium hydroxide and potassium hydroxide. Among those, in a case where potassium hydroxide is used, the acid extract is to contain potassium ions. Potassium ions are important nutrients for plants. Therefore, it is preferable to neutralize the acid extract with potassium hydroxide.

In step S5, bone tissue which has been treated with an acid is treated with a protease. A treatment liquid obtained through step S5 is a protease treatment liquid. Production of a fertilizer from the protease treatment liquid corresponds to the foregoing step 3. Treatment of bone tissue with a protease and an acid is as described in the section [1.2] above.

In step S6, the acid extract and the protease treatment liquid are mixed together. Thus, a bone solubilization liquid B is obtained. Production of a fertilizer from the bone solubilization liquid B corresponds to the foregoing step 4. Preparation of the bone solubilization liquid B is as described in the section [1.3] above.

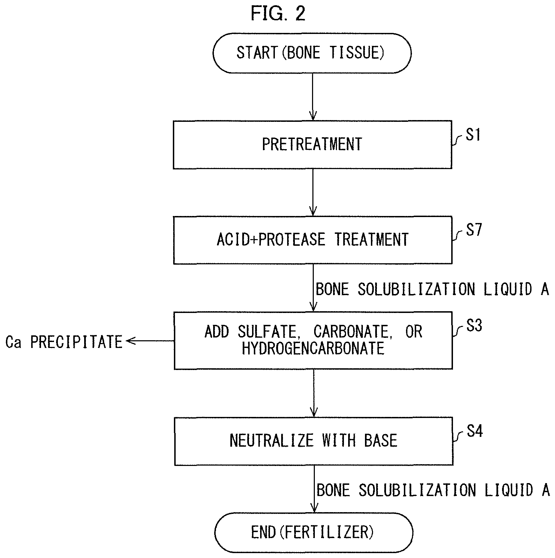

Fig. 2 is an example flowchart illustrating a production method including step 1. In this flowchart, a bone solubilization liquid A is obtained by carrying out step S1, step S7, step S3, and step S4. The bone solubilization liquid A can be used as a component of a fertilizer or a mixed fertilizer.

Steps S1, S3, and S4 are as described above, and therefore descriptions thereof will not be repeated.

In step S7, bone tissue is treated with both an acid and a protease. Thus, a bone solubilization liquid A is obtained. Production of a fertilizer from the bone solubilization liquid A corresponds to the foregoing step 1. Preparation of the bone solubilization liquid A is as described in the section [1.4] above.

### [2. Fertilizer]

### [2.1. Fertilizer specified by production method]

The fertilizer in accordance with an aspect of the present invention is a fertilizer which is obtained by the production method for producing a fertilizer in accordance with an aspect of the present invention. Therefore, the fertilizer in accordance with an aspect of the present invention contains an acid extract, a protease treatment liquid, a bone solubilization liquid A, or a bone solubilization liquid B. Among those, a fertilizer containing a bone solubilization liquid A or a bone solubilization liquid B is preferable because such a fertilizer brings about a higher effect of promoting growth of plant bodies.

A lower limit of a ratio of the acid extract, the protease treatment liquid, the bone solubilization liquid A, or the bone solubilization liquid B accounting for a total weight of the fertilizer can be not less than 0.01% by weight, not less than 0.05% by weight, not less than 0.1% by weight, not less than 0.5% by weight, not less than 1% by weight, not less than 5% by weight, not less than 10% by weight, not less than 20% by weight, not less than 30% by weight, not less than 40% by weight, not less than 50% by weight, not less than 60% by weight, not less than 70% by weight, not less than 80% by weight, or not less than 90% by weight. An upper limit of a ratio of the acid extract, the protease treatment liquid, the bone solubilization liquid A, or the bone solubilization liquid B accounting for a total weight of the fertilizer is 100% by weight. In an embodiment, the fertilizer consists only of an acid extract, a protease treatment liquid, a bone solubilization liquid A, a bone solubilization liquid B, or an arbitrary mixture thereof.

A composition of the fertilizer may be changed as appropriate according to need. For example, a part of or all of phosphorus and calcium may be changed to calcium dihydrogenphosphate or calcium citrate. With such a composition, a fertilizer effect time can be adjusted so that phosphorus and calcium are supplied in accordance with a plant body growth stage.

The fertilizer may be a solid fertilizer or may be a liquid fertilizer. The acid extract, the protease treatment liquid, the bone solubilization liquid A, or the bone solubilization liquid B is obtained in the form of liquid, and therefore can be easily made into a liquid fertilizer. According to the production method for producing a fertilizer in accordance with an embodiment of the present invention, components of bone tissue (e.g., all components of bone tissue) can be solubilized. Therefore, it is expected that a liquid fertilizer rich in components useful for plant growth can be obtained. An existing bone-derived solid fertilizer (bone meal) is slow-acting, and is often used for soil improvement over several years. In contrast, the liquid fertilizer can be expected to be fast-acting. Furthermore, bone meal is not suitable for hydroponic culture, whereas the liquid fertilizer can be suitably used for hydroponic culture. In addition, the liquid fertilizer has an advantage that the liquid fertilizer can be easily applied to foliar spray.

The fertilizer may contain a component other than the acid extract, the protease treatment liquid, the bone solubilization liquid A, or the bone solubilization liquid B. Examples of such a component include an acidic fertilizer, an alkaline fertilizer, and other common fertilizers. Examples of the acidic fertilizer include ammonium sulfate, superphosphate of lime, potassium sulfate, aluminum sulfate, peat moss, black soil, ash, and alum. Examples of the alkaline fertilizer include plant ash, lime nitrogen, Chile niter, fish manure, leaf mold, magnesia lime, organic lime, slaked lime, lime nitrogen, limestone, cement, sodium bicarbonate, shells, chaff, and chaff charcoal. Examples of other common fertilizers include straw, bark, and molasses. The fertilizer may further contain free amino acid (such as γ-aminobutyric acid), plant growth hormone (such as auxin), and trace elements (such as magnesium, sulfur, iron, manganese, zinc, copper, boron, and molybdenum).

### [2.2. Fertilizer specified by components]

The fertilizer in accordance with an aspect of the present invention contains a bone tissue degradation product. Therefore, the fertilizer often contains type I collagen, which is a major organic component of bone tissue, osteocalcin, alpha-2-HS-glycoprotein, periostin, biglycan, SPARC, or a degraded peptide thereof. These components are unlikely to be contained in fertilizers obtained by other production methods. Therefore, a fertilizer containing type I collagen, osteocalcin, alpha-2-HS-glycoprotein, biglycan, SPARC, a degraded peptide thereof, or a combination thereof is highly probably a fertilizer produced by the production method in accordance with an embodiment of the present invention.

In an embodiment, the fertilizer contains a peptide fragment derived from at least one selected from the group consisting of type I collagen, alpha-2-HS-glycoprotein, periostin, biglycan, and SPARC. A production method for producing a fertilizer in accordance with an embodiment of the present invention may include protease treatment of bone tissue (step S5 and step S7). A position of a peptide chain where the peptide chain is cleaved by a protease is determined uniquely by the protease. Therefore, treatment of a specific protein with a specific protease uniquely defines a peptide fragment which is generated by the treatment. Each fragment has a different molecular weight. Therefore, for example, according to mass spectrometry, a protein before protease treatment can be identified from a peptide fragment contained in a fertilizer. Therefore, a person skilled in the art can determine whether or not a peptide fragment contained in a fertilizer is a peptide fragment derived from at least one selected from the group consisting of type I collagen, alpha-2-HS-glycoprotein, periostin, biglycan, and SPARC.

Table 1 shows examples of peptide fragments which can be detected by LC-MS/MS. Each of the peptide fragments is a peptide fragment which can appear when the fertilizer is analyzed without trypsin treatment. Since the trypsin treatment is not carried out, the C-terminus of the peptide fragment is an amino acid that is not Lys or Arg.

### [Table 1]

**Table 1**

| Protein before degradation | Peptide fragment | Molecular weight | SEQ ID No |
|---|---|---|---|
| Type I collagen | GIAGPPG | 568 | 13 |
| | AGLPGVAGAPG | 866 | 14 |
| | PGAPGPKGELGPVG | 1,232 | 15 |
| α-2-HS-glycoprotein | DVPAASLVVGP | 1,024 | 16 |
| | AGADAGATPVVD | 1,043 | 17 |
| | AQPSIPAADGSVPV | 1,308 | 18 |
| Periostin | SALRPDGEYTLL | 1,334 | 19 |
| | GCPAVLPIDH | 1,067 | 20 |
| Biglycan | KDLPETLNEL | 1,171 | 21 |
| SPARC | GIKEQDIDKDL | 1,273 | 22 |
| | DQHPIDGYLS | 1,143 | 23 |

In an embodiment, the peptide fragment derived from at least one selected from the group consisting of type I collagen, alpha-2-HS-glycoprotein, periostin, biglycan, and SPARC does not have physiological activity. The production method for producing a fertilizer in accordance with an embodiment of the present invention includes a plurality of steps in which a protein can lose physiological activity. One of such steps is a pretreatment step. In the pretreatment step, bone tissue is heated, bone tissue is heated under pressure, or bone tissue is irradiated with microwaves. Therefore, a protein is denatured and loses physiological activity. Another one of such steps is protease treatment in the step 1 and the step 3. A protein treated with a protease forms a fragment and loses its original physiological activity.

In an embodiment, a size of the peptide fragment derived from at least one selected from the group consisting of type I collagen, alpha-2-HS-glycoprotein, periostin, biglycan, and SPARC is 10,000 Da or less, 8,000 Da or less, 6,000 Da or less, or 4,000 Da or less. A peptide fragment which has been cleaved to such a size has highly probably lost physiological activity. Moreover, a peptide fragment which has been cleaved to such a size is easily absorbed by a plant as a nutrient, and may function as an active ingredient of a fertilizer.

Herein, "physiological activity of type I collagen, alpha-2-HS-glycoprotein, periostin, biglycan, and SPARC" is as follows. A state in which a fragment derived from the above components has lost physiological activity means that the fragment does not have the following activity.
- Physiological activity of type I collagen: Self-association to form fibers under physiological conditions.
- Physiological activity of alpha-2-HS-glycoprotein: To bind to calcium ions.
- Physiological activity of periostin: To function as a cell adhesion molecule for an osteoblastic progenitor cell.
- Physiological activity of biglycan: To bind to type I collagen.
- Physiological activity of SPARC: To promote type I collagen synthesis of cultured dermal fibroblasts.

In an embodiment, the fertilizer contains phosphoric acid. In the production method for producing a fertilizer in accordance with an embodiment of the present invention, in a case where the pretreatment step is carried out, a concentration of phosphoric acid contained in the fertilizer tends to be high. The concentration of phosphoric acid contained in the fertilizer can be, for example, 280 mM or more, 300 mM or more, or 320 mM or more.

In an embodiment, the fertilizer contains both phosphoric acid and a peptide fragment derived from at least one selected from the group consisting of type I collagen, alpha-2-HS-glycoprotein, periostin, biglycan, and SPARC.

### [3. Production method for producing phosphoric acid]

Another aspect of the present invention relates to a production method for producing phosphoric acid using bone tissue as a raw material. The production method for producing phosphoric acid includes, for example, a step of purifying calcium phosphate and a step of removing calcium.

An example of the step of generating calcium phosphate includes the following procedure.
1. To the acid extract obtained in the step 2, an alkaline solution is added for neutralization. Thus, a precipitate is obtained.
2. To the precipitate, an acid solution (such as hydrochloric acid aqueous solution) is added to solubilize the precipitate again.
3. The above processes 1 and 2 are repeated, and thus calcium phosphate is purified.

In the calcium removal step, calcium is removed by adding a chelating agent to the purified calcium phosphate solution. As another example, to the purified calcium phosphate solution, at least one selected from the group consisting of sulfate, carbonate, and hydrogen carbonate may be added so that calcium is precipitated as calcium sulfate or calcium carbonate. Examples of the sulfate, carbonate, and hydrogen carbonate include sodium sulfate, potassium sulfate, ammonium sulfate, magnesium sulfate, sodium carbonate, sodium hydrogen carbonate, potassium carbonate, potassium hydrogen carbonate, ammonium carbonate, and a mixture of any of these components. A reagent added to the calcium phosphate solution may be added in the form of powder or in the form of solution (such as aqueous solution). The above described method can be applied to an acidic solution and also to room temperature. Therefore, it is possible to simply remove calcium.

### [3.1. Example production method for producing phosphoric acid]

As a method for producing phosphoric acid from bone tissue, two types of production methods are considered. One is a production method in which a bone is incinerated to obtain bone ash, and phosphoric acid is purified from the bone ash. In the step of purifying phosphoric acid from bone ash, a conventional method of purifying phosphoric acid from a phosphate rock is employed. However, this production method consumes a large amount of energy, and a large amount of CO₂ is produced. Therefore, this production method is unsuitable for achieving SDGs.

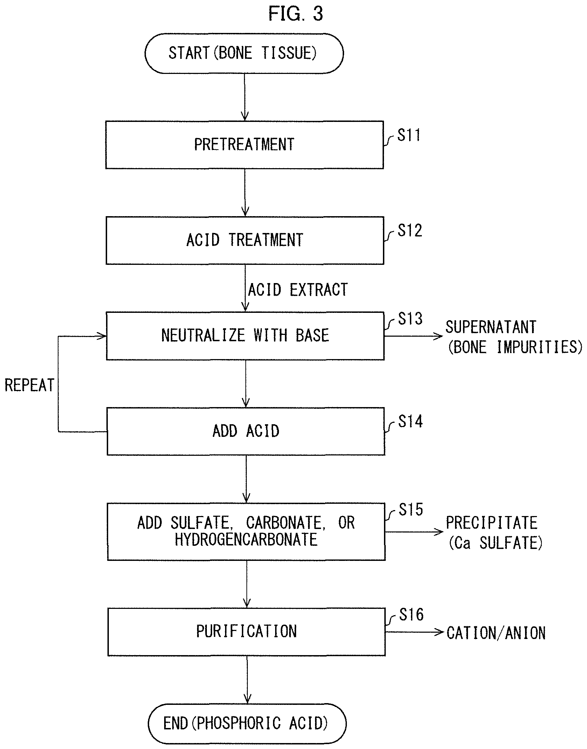

The other production method is a method exemplified in a flowchart of Fig. 3. In addition to phosphoric acid, bone tissue contains inorganic components such as calcium and organic components such as collagen. In the method exemplified in Fig. 3, phosphoric acid is dissolved in step S11 and step S12, and components other than phosphoric acid and calcium are removed in step S13 and step S14. Step S13 and step S14 may be carried out repeatedly, and the number of times of repeat can be set as appropriate. In step S15, sulfate, carbonate, or hydrogen carbonate is added to remove calcium. Calcium sulfate, calcium carbonate, or calcium hydrogen carbonate generated in step S15 may be used as a fertilizer for agriculture or an industrial raw material. The following description will discuss details of the steps.

In step S11, bone tissue is pretreated. Step S11 is an optional step, and does not need to be carried out. An amount of phosphoric acid contained in an acid extract can be increased by pretreating bone tissue. This step is the same step as step S1 described above, and details are as described in the section [1.5] above.

In step S12, bone tissue is treated with an acid. By this step, an acid extract is obtained. This step is the same step as step S2 described above and, for the conditions of acid treatment, descriptions in the section [1.1] above can be applied.

In step S13, the acid extract is neutralized with a base. By step S13, phosphate ions contained in the acid extract precipitate as calcium phosphate, and a component derived from bone impurities is contained in a supernatant. Therefore, by collecting the precipitate, phosphoric acid can be purified. Examples of a base which can be used include sodium hydroxide and potassium hydroxide.

In step S14, an acid is added to the precipitate of calcium phosphate. Thus, calcium phosphate which had been deposited as a precipitate is dissolved again. Examples of the acid which can be used include hydrochloric acid, nitric acid, and formic acid.

Step S13 and S14 may be carried out repeatedly. By repeating these steps, purity of phosphoric acid is increased. The number of times of repeating steps S13 and S14 can be, for example, 2 times or more, 3 times or more, 4 times or more, or 5 times or more. From an economical viewpoint, the number of times steps S13 and S14 are repeated may be, for example, 10 times or less. After repeating steps S13 and S14, before proceeding to step S15, cleaning treatment in which an unnecessary component contained in the calcium phosphate precipitate is cleaned off with pure water may be carried out as many times as necessary.

In step S15, sulfate, carbonate, or hydrogen carbonate is added to the calcium phosphate solution. Thus, calcium ions in the solution precipitate as calcium sulfate, calcium carbonate, or calcium hydrogen carbonate. The precipitated calcium sulfate, calcium carbonate, or calcium hydrogen carbonate is removed by centrifugal separation or the like. An example of sulfate can be at least one selected from sodium sulfate, potassium sulfate, ammonium sulfate, and magnesium sulfate. An example of carbonate can be at least one selected from sodium carbonate, potassium carbonate, and ammonium carbonate. An example of hydrogen carbonate can be at least one selected from sodium hydrogen carbonate and potassium hydrogen carbonate. It is also possible to arbitrarily combine at least two of sulfate, carbonate, and hydrogen carbonate. In step S15, addition of sulfate instead of sulfuric acid brings about the following advantages:
- An increase in volume of the reaction system can be suppressed because a liquid is not added.
- The reaction system does not become strongly acidic and therefore an amount of a base necessary for neutralization can be small.
- Highly safe.
- A removal rate of calcium is high.

An amount of sulfate, carbonate, or hydrogen carbonate added in step S15 can be set as appropriate by a person skilled in the art. An introduction amount of sulfate, carbonate, or hydrogen carbonate may be, for example, an amount with which a concentration of the sulfate, carbonate, or hydrogen carbonate in the reaction system is 0.2 M or more, 0.4 M or more, 0.6 M or more, 0.8 M or more, or 1.0 M or more. A larger introduction amount of sulfate, carbonate, or hydrogen carbonate can reduce residual calcium. An upper limit of the introduction amount of the sulfate, carbonate, or hydrogen carbonate may be, for example, an amount with which a concentration of the sulfate in the reaction system is 3.0 M or less, 2.0 M or less, or 1.0 M.

In step S15, a small amount of sulfate ions, carbonate ions, or hydrogen carbonate ions remains in the supernatant because of a solubility product. In removing these ions, carbonate ions or hydrogen carbonate ions can be easily removed because those ions are converted to carbon dioxide by heating. Therefore, a salt added in step S15 is preferably at least one selected from the group consisting of carbonate and hydrogen carbonate.

In step S16, a supernatant from which calcium has been removed is purified. The supernatant may contain cations such as sodium ions contained in sulfate and anions such as chloride ions. Highly pure phosphoric acid is obtained by causing, for example, an ion exchange resin to adsorb those ions. In a case where carbonate ions or hydrogen carbonate ions are contained, ions can be removed from the system by converting the ions to carbon dioxide by heating.

The present invention is not limited to the embodiments described above, but may be altered in various ways by a skilled person within the scope of the claims. Any embodiment based on a proper combination of technical means disclosed in different embodiments is also encompassed in the technical scope of the present invention.

### Examples

### [Example 1: Preparation of fertilizer containing bone tissue degradation product]

### [Example 1-1: Preparation of acid extract by nitric acid and component analysis]

Bone tissue was treated with nitric acid with a procedure described below, and thus an acid extract was obtained.
1. A pig bone obtained from a slaughterhouse was finely pulverized with a mill (IKA TUBE MILL 100, IKA JAPAN K.K.).
2. To the pig bone in a wet weight of 3 g, 40 mL of a nitric acid aqueous solution was added, and the pig bone was immersed in the aqueous solution at 20°C. A concentration of the nitric acid aqueous solution was 0.3 mol/L, 0.5 mol/L, 0.75 mol/L, or 1.0 mol/L. An immersion time was 12 hours, 24 hours, or 48 hours.
3. A supernatant was collected, and thus an acid extract was obtained.

### [Example 1-2: Preparation of acid extract by hydrochloric acid and component analysis]

Bone tissue was treated with hydrochloric acid with a procedure described below, and thus an acid extract was obtained.
1. A pig bone obtained from a slaughterhouse was finely pulverized with a mill (IKA TUBE MILL 100, IKA JAPAN K.K.).
2. To the pig bone in a wet weight of 2 g, 35 mL of a hydrochloric acid aqueous solution was added, and the pig bone was immersed in the aqueous solution at 20°C. A concentration of the hydrochloric acid aqueous solution was 0.3 mol/L, 0.5 mol/L, or 1.0 mol/L. An immersion time was 6 hours, 12 hours, or 24 hours.
3. A supernatant was collected, and thus an acid extract was obtained.

### [Example 1-3: Preparation of acid extract by formic acid and component analysis]

Bone tissue was treated with formic acid with a procedure described below, and thus an acid extract was obtained.
1. A pig bone obtained from a slaughterhouse was finely pulverized with a mill (IKA TUBE MILL 100, IKA JAPAN K.K.).
2. To the pig bone in a wet weight of 2 g, 35 mL of a formic acid aqueous solution was added, and the pig bone was immersed in the aqueous solution at 20°C. A concentration of the formic acid aqueous solution was 0.3 mol/L, 0.5 mol/L, or 1.0 mol/L. An immersion time was 6 hours, 12 hours, or 24 hours.
3. A supernatant was collected, and thus an acid extract was obtained.

### [Example 1-4: Preparation of acid extract by sulfuric acid and component analysis]

Bone tissue was treated with sulfuric acid with a procedure described below, and thus an acid extract was obtained.
1. A pig bone obtained from a slaughterhouse was finely pulverized with a mill (IKA TUBE MILL 100, IKA JAPAN K.K.).
2. To the pig bone in a wet weight of 2 g, 35 mL of a sulfuric acid aqueous solution was added, and the pig bone was immersed in the aqueous solution at 20°C. A concentration of the sulfuric acid aqueous solution was 0.3 mol/L, 0.5 mol/L, or 1.0 mol/L. An immersion time was 6 hours, 12 hours, or 24 hours.
3. A supernatant was collected, and thus an acid extract was obtained.

Table 2 shows a result of analyzing total phosphorus in the obtained acid extract. The component analysis was outsourced to Kurita Analysis Service Co., Ltd. Table 2 shows a measurement result of total phosphorus (mg) calculated per gram in terms of wet weight of bone. The analysis results were assembled for each acid in terms of concentration of nitric acid, hydrochloric acid, formic acid, or sulfuric acid, and immersion time. It has been shown that most phosphorus can be recovered in approximately 24 hours in all the acid aqueous solutions. It has also been shown that the acid concentration of approximately 1 mol/L can sufficiently recover phosphorus. Among the four types of acids studied, hydrochloric acid and sulfuric acid exhibited high phosphorus recovery efficiency. From Table 2, it has been found that approximately 100 mg of phosphorus can be recovered from 1 g of bone tissue.

### [Table 2]

**Table 2**

| Nitric acid aqueous solution | | | | Hydrochloric acid aqueous solution | | | |
|---|---|---|---|---|---|---|---|
| Concentration (mol/L) | Immersion time(h) | | | Concentration (mol/L) | Immersion time(h) | | |
| | 12 | 24 | 48 | | 6 | 12 | 24 |
| 0.5 | 70 | 74 | 74 | 0.3 | 19 | 62 | 60 |
| 0.75 | 84 | 77 | 84 | 0.5 | 64 | 89 | 87 |
| 1.0 | 81 | 88 | 88 | 1.0 | 98 | 107 | 107 |

| Formic acid aqueous solution | | | | Sulfuric acid aqueous solution | | | |
|---|---|---|---|---|---|---|---|
| Concentration (mol/L) | Immersion time(h) | | | Concentration (mol/L) | Immersion time(h) | | |
| | 6 | 12 | 24 | | 6 | 12 | 24 |
| 0.5 | 65 | 47 | 55 | 0.5 | 83 | 77 | 102 |
| 0.75 | 74 | 70 | 77 | 0.75 | 89 | 95 | 100 |
| 1.0 | 74 | 86 | 79 | 1.0 | 91 | 74 | 105 |

Table 3 shows a result of analyzing total calcium in the obtained acid extract. The component analysis was outsourced to Kurita Analysis Service Co., Ltd. Table 3 shows a measurement result of total calcium (mg) calculated per gram in terms of wet weight of bone. The analysis results were assembled for each acid in terms of concentration of nitric acid, hydrochloric acid, formic acid, or sulfuric acid, and immersion time. It has been shown that most calcium can be recovered in approximately 24 hours in the aqueous solutions of nitric acid, hydrochloric acid, and formic acid. In the sulfuric acid aqueous solution, free calcium forms insoluble calcium sulfate. Therefore, as a result of the analysis, the calcium concentration is low but calcium is actually eluted from the bone tissue. It has also been shown that the acid concentration of approximately 1 mol/L can sufficiently recover calcium. Among the four types of acids studied, hydrochloric acid and formic acid exhibited high calcium recovery efficiency. From Table 3, it has been found that approximately 200 mg of calcium can be recovered from 1 g of bone tissue.

### [Table 3]

**Table 3**

| Nitric acid aqueous solution | | | | Hydrochloric acid aqueous solution | | | |
|---|---|---|---|---|---|---|---|
| Concentration (mol/L) | Immersion time(h) | | | Concentration (mol/L) | Immersion time(h) | | |
| | 12 | 24 | 48 | | 6 | 12 | 24 |
| 0.5 | 151 | 136 | 149 | 0.3 | 35 | 107 | 107 |
| 0.75 | 165 | 152 | 162 | 0.5 | 116 | 161 | 170 |
| 1.0 | 165 | 164 | 179 | 1.0 | 179 | 196 | 214 |

| Formic acid aqueous solution | | | | Sulfuric acid aqueous solution | | | |
|---|---|---|---|---|---|---|---|
| Concentration (mol/L) | Immersion time(h) | | | Concentration (mol/L) | Immersion time(h) | | |
| | 6 | 12 | 24 | | 6 | 12 | 24 |
| 0.5 | 120 | 164 | 183 | 0.5 | 13 | 11 | 12 |
| 0.75 | 152 | 194 | 205 | 0.75 | 11 | 11 | 12 |
| 1.0 | 192 | 182 | 192 | 1.0 | 11 | 11 | 14 |

### [Example 1-5: Preparation of protease treatment liquid]

The bone tissue which had been treated with an acid in Example 1-2 was immersed in a treatment liquid containing two types of proteases below. Conditions of protease treatment were as follows. Protease concentration: 2% (w/w), temperature: 50°C, pH: optimum pH.
- Protease 1: Newlase F3G (Amano Enzyme Inc., protease derived from filamentous fungus Rhizopus niveus)
- Protease 2: pepsin (Sigma Aldrich, aspartic protease)

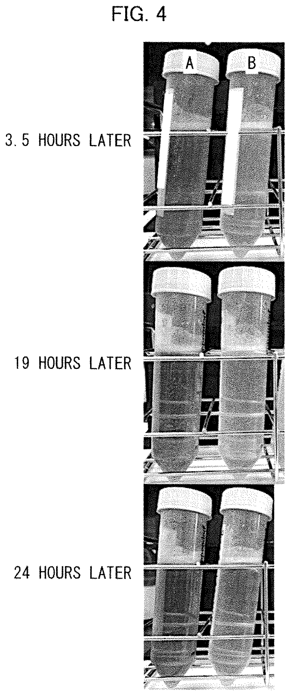

Results are shown in Fig. 4. In Fig. 4, "A" represents a result of treatment with the protease 1, and "B" represents a result of treatment with the protease 2. A treatment time was 3.5 hours, 19 hours, or 24 hours. Results of visually checking transparency of the treatment liquid and remaining bone tissue were as follows.
- After 3.5 hours: In all of the protease-treated systems, bone tissue was almost lost.
- After 24 hours: In all of the protease-treated systems, bone tissue was completely lost, and transparency of the protease treatment liquid was increased.

It has been thus found that a protease treatment liquid can be obtained by treating, with a commercially available protease, bone tissue which has been treated with an acid.

### [Example 1-6: Preparation of protease treatment liquid and component analysis]

A protease treatment liquid was obtained with a procedure below.
1. Bone tissue was treated with an acid by being immersed in a nitric acid aqueous solution or a hydrochloric acid aqueous solution of 1 mol/L for 48 hours.
2. A resultant treatment liquid was sorted out into an acid extract and a bone tissue residue, and the acid extract was transferred to another container.
3. To the bone tissue residue, a citrate buffer solution (pH: 3.5) of 0.1 mol/L was added. The added amount was 10 mL per gram in terms of initial bone weight.
4. Actinidain (cysteine protease derived from a kiwi fruit) was pretreated. Specifically, the actinidain was brought into contact with a 20 mmol/L phosphate buffer solution (pH: 6.5) containing 10 mmol/L of dithiothreitol and 5 mmol/L of ethylenediaminetetraacetic acid for 90 minutes at 25°C.
5. To a reaction system obtained in the above process 3, activated actinidain was added to carry out protease treatment. Treatment conditions were as follows. Temperature: 20°C or 50°C, pH: 3.5 (adjusted with 100 mmol/L phosphate buffer solution), time: 3 days.

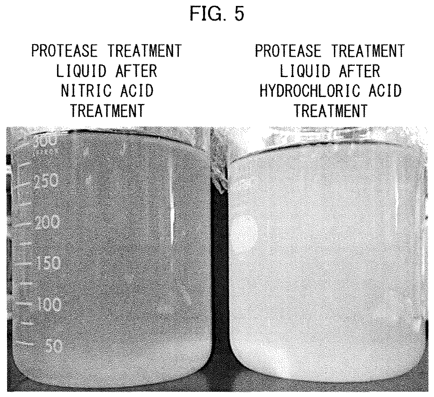

Results are shown in Fig. 5. In the system in which the treatment was carried out with either nitric acid or hydrochloric acid, bone tissue was almost lost when the reaction temperature was 50°C.

The obtained protease treatment liquid was passed through a filter of 100 um, and a filtrate from which a fine insoluble substance had been removed was obtained. Results of component analysis of the filtrate are shown in Table 4 (numerical values calculated per gram in terms of wet weight of bone are shown). The component analysis was outsourced to Kurita Analysis Service Co., Ltd.

### [Table 4]

**Table 4**

| | | | | |
|---|---|---|---|---|
| Acid treatment | 1M nitric acid | | 1M hydrochloric acid | |
| Protease treatment | Actinidain | | | |
| Reaction temperature(°C) | 20 | 50 | 20 | 50 |
| Electric conductivity(S/m) | 20.2 | 1.21 | ND | ND |
| Total nitrogen(g/L) | 19.2 | 1.72 | 0.78 | 2.23 |
| Total phosphorus(mg/L) | 22.2 | 384 | 212 | 186 |
| Potass ium (mg/L) | 8.08 | 10.1 | ND | ND |
| Total calcium(mg/L) | <20.2 | 313 | ND | ND |
| Total magnesium(mg/L) | <505 | 5.1 | ND | ND |

| | | | | |
|---|---|---|---|---|
| ND:Not done | | | | |

### [Example 1-7: Preparation of bone solubilization liquid A and component analysis]

A bone solubilization liquid A was prepared in one stage without involving solution replacement between the acid treatment and the protease treatment. A specific procedure was as follows.
1. Bone tissue was treated with an acid by being immersed in a nitric acid aqueous solution, a hydrochloric acid aqueous solution, a formic acid aqueous solution, or a sulfuric acid aqueous solution, each of which was of 1 mol/L, for 48 hours.
2. To a reaction system, a citrate buffer solution (pH: 3.5) was added so that a final concentration was 0.1 mol/L.
3. With 5N of NaOH and 5N of HCl, a pH was adjusted to an optimum pH of a protease. Specifically, actinidain was adjusted to have a pH of 3.5, pepsin was adjusted to have a pH of 3.0, and Newlase F3G was adjusted to have a pH of 3.0.
4. Each of the proteases was added to be 1% (w/w). Reaction conditions of the protease were as follows: a temperature of 50°C for 4 days. Note that the actinidain (cysteine protease derived from a kiwi fruit) was pretreated at 25°C for 90 minutes with a 20 mmol/L phosphate buffer solution (pH: 6.5) containing 10 mmol/L of dithiothreitol and 5 mmol/L of ethylenediaminetetraacetic acid.

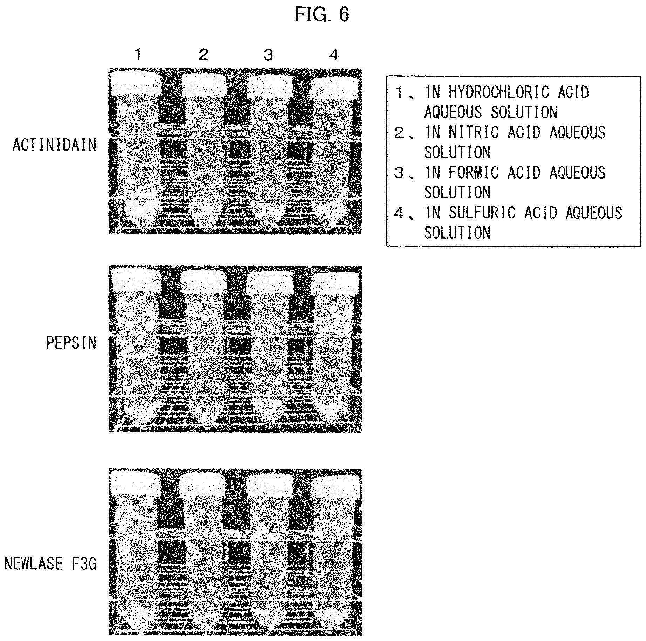

Results are shown in Fig. 6. In all the systems, when the reaction temperature was 50°C, the bone tissue was reduced in size. Thus, a bone solubilization liquid A was obtained from bone tissue in one-step manner without carrying out solution replacement between the acid treatment and the protease treatment. In particular, the combination of pepsin with hydrochloric acid or nitric acid well dissolved bone tissue, and only few remaining bone tissue was found.

The obtained bone solubilization liquid A was passed through a filter of 100 µm, and a filtrate from which a fine insoluble substance had been removed was obtained. Results of measuring the calcium concentration in the filtrate are shown in Table 5 (numerical values calculated per gram in terms of wet weight of bone are shown). In measurement of calcium, a compact calcium ion meter LAQUAtwin-Ca-11 (HORIBA Advanced Techno, Co., Ltd.) was used.

### [Table 5]

**Table 5**

| Immersion time(96h) | |
|---|---|
| Acid | Calcium (mg) |
| Hydrochloric acid aqueous solution | 226 |
| Nitric acid aqueous solution | 238 |
| Formic acid aqueous solution | 133 |
| Sulfuric acid aqueous solution | 97 |

### [Example 1-8: Extraction of phosphoric acid]

With a procedure below, roughly purified phosphoric acid was obtained from bone tissue.
1. Bone tissue was treated with an acid by being immersed in a nitric acid aqueous solution, a hydrochloric acid aqueous solution, a formic acid aqueous solution, or a sulfuric acid aqueous solution, each of which was of 1 mol/L, for 48 hours.
2. To a reaction system, 5N of NaOH was added by a capacity of 0.15 and mixed well, and a resultant mixture was left to stand still at room temperature for 1 hour. Thus, a white solution was obtained.
3. The obtained solution was subjected to centrifugal separation at 10,000 g, for 10 minutes, and at room temperature to remove a supernatant. To a white precipitate, 1N of HCl was added by an amount identical with that added in the step 1, and a resultant mixture was mixed well. Thus, a transparent solution was obtained.
4. Na₂SO₄, K₂SO₄, MgSO₄, (NH₄)₂SO₄, Na₂CO₃, or NaHCO₃ was added in an amount with which a concentration thereof was identical with a calcium concentration in the reaction system and was mixed well, and a resultant mixture was left to stand still at room temperature for 1 hour. Thus, a white solution was obtained.
5. The obtained solution was subjected to centrifugal separation at 10,000 g, for 10 minutes, and at room temperature to collect a supernatant. A resultant transparent solution contained phosphoric acid.

By immersing bone tissue in sulfuric acid, it is possible to obtain a phosphoric acid-containing solution in which calcium has been removed to a certain extent. However, in treatment using sulfuric acid, a capacity increases, a solution becomes strongly acidic, an amount of impurities is large, and thus purification is difficult. In contrast, according to the above described steps, it is possible to simply obtain a solution containing phosphoric acid with high purity.

### [Example 2: Growth evaluation of plant body cultivated with fertilizer containing bone tissue degradation product and component analysis]

### [Materials and method]

1. A seed of radish (Raphanus sativus L) (TAKII & CO., LTD.) was sowed on a wet paper towel. The seed was left to stand still at 22°C for 2 days under a dark condition, and thus the seed was caused to sprout.
2. A resultant plant body was transplanted to a urethane cube (2 cm × 2 cm × 2 cm) for hydroponic culture.
3. The urethane cube was immersed in an acid extract, a protease treatment liquid, or a bone solubilization liquid A, and growing was carried out for 5 days. Light conditions during the period were as follows. Photosynthetically active photon flux density: approximately 150 µmol/m²·s, light period: 16 hours, dark period: 8 hours. As a light source, a fluorescent lamp (BIOLUX A, NEC Lighting, Ltd.) for growing a domesticated plant was used.
4. Growth evaluation and component analysis for the grown plant body were carried out. Specifically, the following processes were carried out.
   - Above-ground fresh weight: The plant body was cleaved at the boundary between the hypocotyl and the root, and a weight on the hypocotyl side was measured.
   - Above-ground dry weight: A part of the plant body for which the fresh weight was measured was dried in an oven at 80°C for 2 days. After that, a weight was measured.
   - Above-ground moisture content: Calculated based on a difference between the above-ground fresh weight and the above-ground dry weight.
   - Leaf area: The cotyledon of the plant body was scanned by a scanner, and a leaf area was measured using Imaged.
   - Total polyphenol content: 50 mg of the cotyledon was crushed in a 90% methanol solution. Using a supernatant obtained by centrifugal separation, a total polyphenol content was measured by the Folin-Ciocalteu method.

### [Results and consideration]

### (1) Influence of acid extract concentration on growth

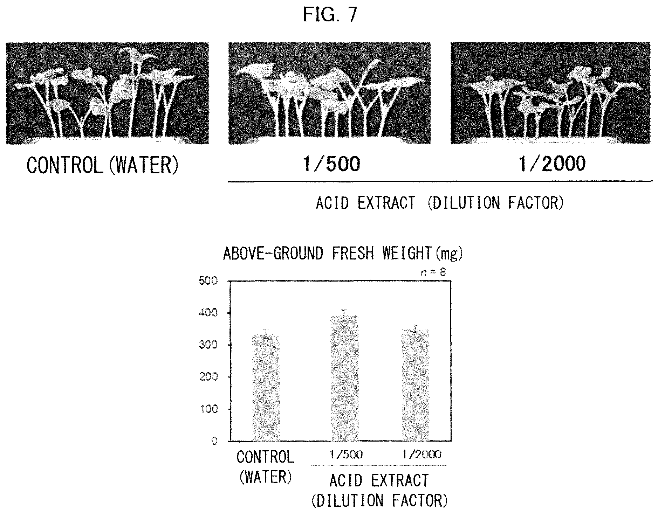

A plant body was grown under conditions where a 500-fold diluted acid extract or 2000-fold diluted acid extract was provided. Results are shown in Fig. 7. The plant body which was grown with the 500-fold diluted acid extract showed an increase in above-ground fresh weight, as compared with a control plant body. From the result, it has been found that the acid extract diluted approximately 500 times has a growth-promoting effect on the plant body. In subsequent experiments, an acid extract which was diluted 500 times and a protease treatment liquid were used.

### (2) Influence of acid extract, protease treatment liquid, or bone solubilization liquid A on growth

Plant bodies were grown under the following six conditions, and above-ground fresh weights were compared.
- Provide only water (control)
- Provide a protease treatment liquid which has not been heated (protease treatment liquid 1)
- Provide a protease treatment liquid which has been heated at 50°C (protease treatment liquid 2)
- Provide an acid extract
- Provide a bone solubilization liquid A in which the protease treatment liquid 1 is mixed with the acid extract
- Provide a bone solubilization liquid A in which the protease treatment liquid 2 is mixed with the acid extract

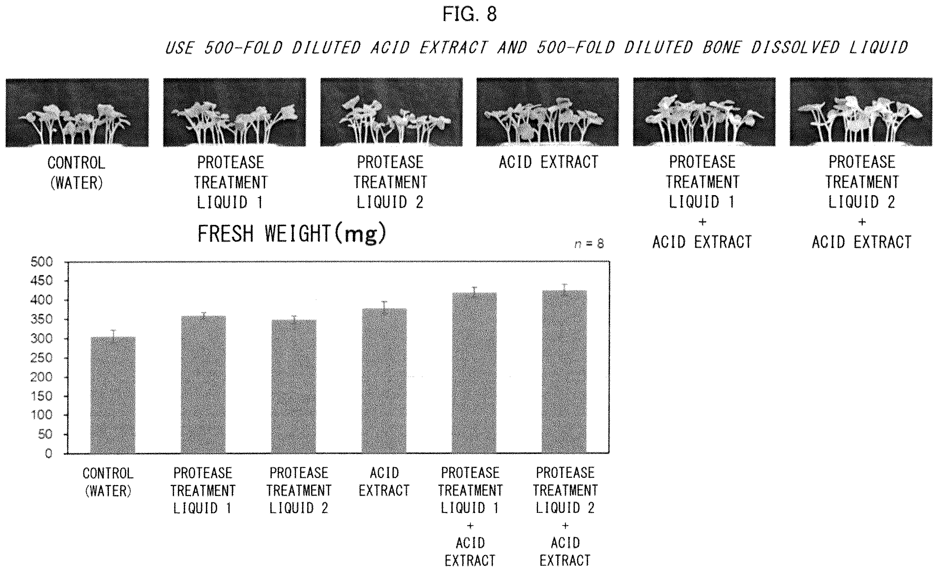

Results are shown in Fig. 8. Growth of the plant bodies provided with the protease treatment liquid 1 and the protease treatment liquid 2 was promoted, as compared with the control. Similarly, growth of the plant body provided with the acid extract was also promoted, as compared with the control (this point is as shown in (1)). Furthermore, the plant body provided with the bone solubilization liquid A in which the protease treatment liquid was mixed with the acid extract had a higher growth-promoting effect, as compared with the control. From the results, it can be seen that both the acid extract and the protease treatment liquid have the growth-promoting effect on plant bodies. Moreover, it has been found that the growth-promoting effect on plant bodies by the bone solubilization liquid A is higher than that by the acid extract alone or the protease treatment liquid alone.

### (3) Comparison of effect with commercially available culture solution

A growth-promoting effect of the bone solubilization liquid A was compared with that of a commercially available culture solution. As the commercially available culture solution, one obtained by further 1/6 dilution of the formulation A of OAT House (OAT Agrio Co., Ltd.) was used. With this dilution, the commercially available culture solution was adjusted to have a nitrate ion concentration (approximately 600 ppm) which was identical with that of the bone solubilization liquid A.

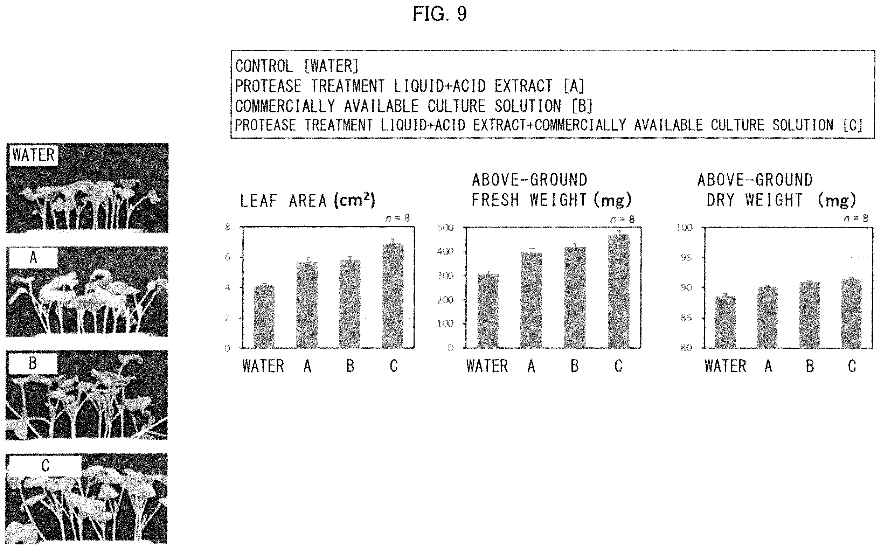

Results are shown in Figs. 8 and 9. Growth of a plant body provided with the commercially available culture solution was promoted to a similar extent as a plant body provided with the bone solubilization liquid A. A plant body provided with both the bone solubilization liquid A and the culture solution showed a further enhanced growth-promoting effect than the plant body provided with each of those alone. Thus, it has been suggested that the growth-promoting effect of the commercially available culture solution can be further improved by the bone solubilization liquid A.

The above-ground moisture content of a plant body was increased by providing the bone solubilization liquid A, and further increased by adding both the bone solubilization liquid A and the culture solution. The total polyphenol content per cotyledon was increased by providing the bone solubilization liquid A, and further increased by providing both the bone solubilization liquid A and the culture solution. This seems to be because of influence caused by an increase in cotyledon weight as a result of promoting plant body growth.

### [Example 3: RNA expression analysis 1 of plant body]

### [Total RNA extraction of plant body]

1. A seed of radish (Raphanus sativus L) (TAKII & CO., LTD.) was sowed on a wet paper towel. The seed was left to stand still at humidity of 100% and at 22°C for 2 days under a dark condition, and thus the seed was caused to sprout.
2. A resultant plant body was transplanted to a urethane sponge. The urethane sponge was impregnated with any of (1) water, (2) a bone solubilization liquid A, (3) a commercially available liquid fertilizer, and (4) a mixed solution of a bone solubilization liquid A and a commercially available liquid fertilizer.
3. The plant body was grown at 22°C for 1 day, 3 days, or 5 days. Light conditions during the period were as follows. Light intensity: approximately 100 µmol/m²·s, light period: 16 hours, dark period: 8 hours. As a light source, a fluorescent lamp (BIOLUX A, NEC Lighting, Ltd.) for growing a domesticated plant was used.
4. From the grown plant body, tissues of a leaf and a root were sampled by approximately 0.1 g each. The tissue was put into a mortar soon after the sampling, liquid nitrogen was added to the mortar, and the tissue in a frozen state was ground.
5. With use of RNeasy Plant Mini Kit (Qiagen), total RNA was extracted from the ground tissue.
6. With use of Qubit RNA HS Assay Kit (Thermo Fisher Scientific) and a fluorophotometer (Qubit-4), a total RNA concentration in the aqueous solution was measured. In addition, absorbances (A₂₃₀, A₂₆₀, and A₂₈₀) in 230 nm, 260 nm, and 280 nm were measured with a spectrophotometer, and purity of extracted RNA was confirmed.

Results are shown in Table 6. From approximately 0.1 g of the plant body leaf, total RNA was extracted in an amount of 13.2 µg to 88.2 µg during a period from the start of culture to day 5. From approximately 0.1 g of the plant body root, 8.4 µg to 43.8 µg of total RNA was extracted. A₂₆₀/A₂₈₀ was approximately 2.0, and it was thus confirmed that there was no problem with RNA purity.

### [Table 6]

**Table 6**

| Culture period | Cultivation condition | Tissue | A230 | A260 | A280 | Dilution factor | Concentration (*µ* g/ *µ* L) | 260/230 | 260/280 | Total amount of RNA (*µ* g) (for 60*µ* L) |
|---|---|---|---|---|---|---|---|---|---|---|
| Day 0 | (1) | Leaf | 0.6 | 1.28 | 0.62 | 25 | 1.28 | 2.13 | 2.06 | 76.8 |
| Day 0 | (1) | Root | 0.28 | 0.73 | 0.37 | 25 | 0.73 | 2.61 | 1.97 | 43.8 |
| Day 1 | (1) | Leaf | 0.36 | 0.78 | 0. 38 | 25 | 0.78 | 2.17 | 2.05 | 46.8 |
| Day 1 | (1) | Root | 0.16 | 0.44 | 0.19 | 25 | 0.44 | 2.75 | 2.32 | 26.4 |
| Day 1 | (2) | Leaf | 0.6 | 1.14 | 0.67 | 25 | 1.14 | 1.90 | 1.70 | 68.4 |
| Day 1 | (2) | Root | 0.17 | 0.47 | 0.2 | 25 | 0.47 | 2.76 | 2.35 | 28.2 |
| Day 1 | (3) | Leaf | 0.51 | 1.22 | 0.59 | 25 | 1.22 | 2.39 | 2.07 | 73.2 |
| Day 1 | (3) | Root | 0.12 | 0.24 | 0.085 | 25 | 0.24 | 2.00 | 2.82 | 14.4 |
| Day 1 | (4) | Leaf | 0.7 | 1.47 | 0.68 | 25 | 1.47 | 2.10 | 2.16 | 88.2 |
| Day 1 | (4) | Root | 0.15 | 0.28 | 0.11 | 25 | 0.28 | 1.87 | 2.55 | 16.8 |
| Day 3 | (1) | Leaf | 0.26 | 0. 65 | 0.3 | 25 | 0.65 | 2.50 | 2.17 | 39.0 |
| Day 3 | (1) | Root | 0.11 | 0.21 | 0.1 | 25 | 0.21 | 1.91 | 2.10 | 12.6 |
| Day 3 | (2) | Leaf | 0.31 | 0.69 | 0.32 | 25 | 0.69 | 2.23 | 2.16 | 41.4 |
| Day 3 | (2) | Root | 0.08 | 0.18 | 0.081 | 25 | 0.18 | 2.25 | 2.22 | 10.8 |
| Day 3 | (3) | Leaf | 0.35 | 0.87 | 0.4 | 25 | 0.87 | 2.49 | 2.18 | 52.2 |
| Day 3 | (3) | Root | 0.17 | 0.22 | 0.1 | 25 | 0.22 | 1.29 | 2.20 | 13.2 |
| Day 3 | (4) | Leaf | 0.38 | 0.91 | 0.41 | 25 | 0.91 | 2.39 | 2.22 | 54.6 |
| Day 3 | (4) | Root | 0.14 | 0.35 | 0.15 | 25 | 0.35 | 2.50 | 2.33 | 21.0 |
| Day 5 | (1) | Leaf | 0.068 | 0. 24 | 0.13 | 25 | 0.24 | 3.53 | 1.85 | 14.4 |
| Day 5 | (1) | Root | 0.033 | 0.14 | 0.062 | 25 | 0.14 | 4.24 | 2.26 | 8.4 |
| Day 5 | (2) | Leaf | 0.065 | 0.22 | 0.11 | 25 | 0.22 | 3.38 | 2.00 | 13.2 |
| Day 5 | (2) | Root | 0.068 | 0.22 | 0.1 | 25 | 0.22 | 3.24 | 2.20 | 13.2 |
| Day 5 | (3) | Leaf | 0.19 | 0.55 | 0.29 | 25 | 0.55 | 2.89 | 1.90 | 33.0 |
| Day 5 | (3) | Root | 0.16 | 0.22 | 0.11 | 25 | 0.22 | 1.38 | 2.00 | 13.2 |
| Day 5 | (4) | Leaf | 0.2 | 0.61 | 0.31 | 25 | 0.61 | 3.05 | 1.97 | 36.6 |
| Day 5 | (4) | Root | 0.22 | 0.3 | 0.16 | 25 | 0.3 | 1.36 | 1.88 | 18.0 |

### [Preparation of RNA sequence library and RNA sequence analysis by next-generation sequencer]

Preparation of a cDNA library from total RNA extracted from the sprout and gene expression analysis by a next-generation sequencer were outsourced to Azenta Japan Corp. A procedure for the analysis was as follows.
1. Quality (degradation degree of RNA) of the extracted total RNA was evaluated by BioAnalyzer (Agilent Technologies).
2. From the extracted total RNA, polyA-mRNA was concentrated using beads of bound polyT oligoDNA.
3. A cDNA library was prepared using a reverse transcriptase.
4. A next-generation sequencer (DNBSEQ-G400, MGI tech) was used to determine a base sequence of the cDNA. Thus, a base sequence of mRNA being expressed was comprehensively determined (RNAseq analysis). Assembly of the sprout gene was carried out by Stringtie software. At that time, a reference was made to a whole-genome base sequence (http://radish.kazusa.or.jp) of a sprout which is opened to public on a database of Kazusa DNA Research Institute.

### [Analysis of gene whose expression amount was changed]

1. Genes whose expression amount was significantly changed by a difference in cultivation conditions were identified based on fragments per kilobase of exon per million reads mapped (FPKM) values which were each obtained by correcting a read number of each gene with a transcript length. In the identification, DESeq2 software was used.
2. A gene (DEG) whose expression amount was significantly increased or decreased was comprehensively determined. The analysis result was indicated by a volcano plot. Comparison of expression amounts was carried out in four groups, i.e., (1) water only, (2) a bone solubilization liquid A, (3) a commercially available liquid fertilizer, and (4) a mixed solution of a bone solubilization liquid A and a commercially available liquid fertilizer. In the group (2) or (4), a gene whose expression amount was significantly increased or decreased was identified.
3. For the identified expression variable genes, GO enrichment analysis was carried out using GOseq software. Thus, biological functions of expression variable genes were analyzed.

### [Quantitative RT-PCR]

Five types of genes which are deeply related to stress tolerance were used as target genes, and expression of the gene was studied by quantitative RT-PCR. As the target genes, glutathione S-transferase τ19 (GSTU19), catalase 2 (CAT2), auxin transporter analogous protein 2-1 (LAX2-1), glutathione S-transferase 12 (GST12), and calmodulin 5 (CaMS) were employed. A specific procedure was as follows.
1. Total RNA was extracted from a leaf and a root of a plant body which had been grown while providing (1) water, (2) a bone solubilization liquid A, (3) a commercially available liquid fertilizer, or (4) a mixed solution of a bone solubilization liquid A and a commercially available liquid fertilizer.
2. Reverse transcriptase reaction (RT reaction) was carried out using a random primer. As a reverse transcriptase, PrimeScript RT Master Mix (Takara Bio Inc.) was used.
3. The target gene was subjected to quantitative PCR. In the quantitative PCR, TB Green Premix Ex Taq II (Takara Bio Inc.), which is a specific primer pair, was used in which 2.0 ng of cDNA was used as a template. As an instrument for carrying out the quantitative PCR, Thermal Cycler Dice Real Time System TP850 (Takara Bio Inc.) was used.

In the quantitative RT-PCR, an expression amount of each target gene was normalized with respect to an expression amount of an actin gene. Base sequences of primer pairs used for amplification of the target genes and actin genes are shown in Table 7 (SEQ ID Nos. 1 through 12 in order from top).

### [Table 7]

**Table 7**

| Base sequence (5' →3' ) | | | |
|---|---|---|---|
| Actin | ACT | F | GCATCACACTTTCTACAAC |
| | | R | CCTGGATAGCAACATACAT |
| glutathione S-transferase TAU 19 | GSTU19 | F | ACTGAACATAGATAGGAACC |
| | | R | GTGGACATTGGATTGATTG |
| catalase 2 | CAT2 | F | GGGCAATAAGCAAATATGAA |
| | | R | GCAACCTGGAGATAGATAC |
| auxin transporter-like protein 2-1 | LAX2-1 | F | GTGGGAGAAAGTGATAGG |
| | | R | ATTGATTGGACCGAAGAA |
| glutathione S-transferase 12 | GST12 | F | GGATGGAATAATGAATGAATGA |
| | | R | AGTTTTGGCACATACAGT |
| calmodulin 5 (Ca2+ binding) | CaM5 | F | CAAAGAACCCATCACAGA |
| | | R | AACAGAGGAAGAGGACAT |

### [Results]

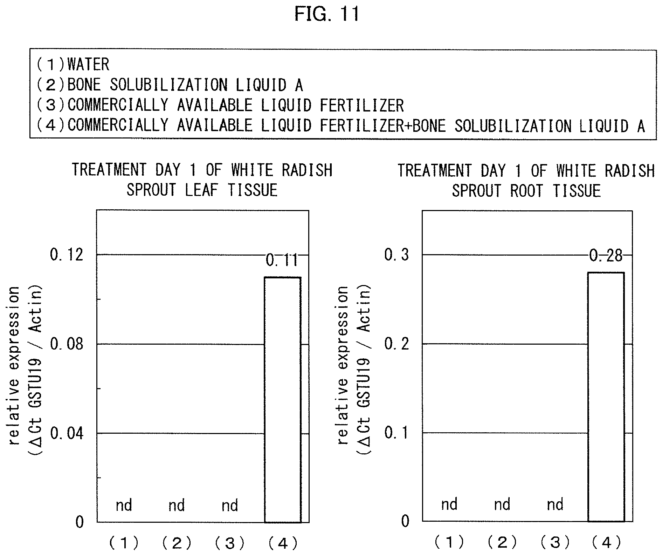

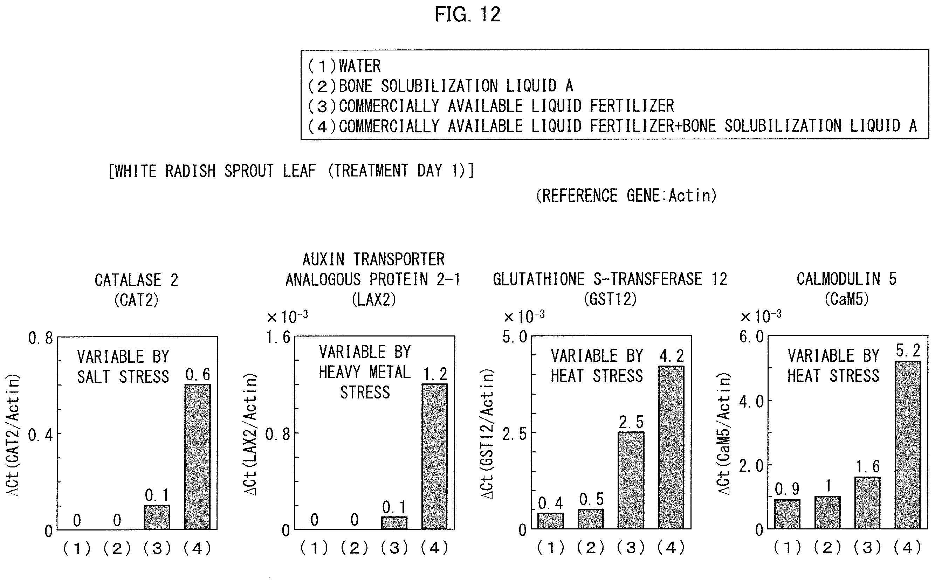

An expression amount of the gene encoding glutathione S-transferase τ19 (GSTU-19) was not detected before transplantation to the urethane sponge. Similarly, at the time point on day 1 after transplantation, the expression amount was not detected in any of leaves and roots of the plant bodies which were grown in systems containing (1) water, (2) a bone solubilization liquid A, and (3) a commercially available liquid fertilizer. However, at the time point on day 1 after transplantation, in the leaf and root of the plant body which was grown in a system containing (4) a mixed solution of a bone solubilization liquid A and a commercially available liquid fertilizer, an expression amount of GSTU-19 was remarkably increased (Fig. 11). Similarly, at the time point on day 1 after transplantation, in the leaf of the plant body which was grown in the system containing (4) a mixed solution of a bone solubilization liquid A and a commercially available liquid fertilizer, expression amounts of genes encoding the catalase 2 (CAT2), the auxin transporter analogous protein 2-1 (LAX2-1), the glutathione S-transferase 12 (GST12), and the calmodulin 5 (CaMS) were remarkably increased (Fig. 12).

It was thus shown that use of the fertilizer in accordance with an embodiment of the present invention in combination with an existing liquid fertilizer promotes expression of genes involved in plant stress tolerance. This result suggests that a combination of the fertilizer in accordance with an embodiment of the present invention and an existing liquid fertilizer can improve stress tolerance of a plant body.

### [Example 4: Improvement of phosphoric acid extraction ratio by pretreatment]

It was confirmed that an amount of phosphoric acid contained in an acid extract increased by pretreating bone tissue. Specifically, by the following procedure, an acid extract was prepared and a contained amount of phosphoric acid was quantitatively determined.
1. A pig bone obtained from a slaughterhouse was finely pulverized with a mill (IKA TUBE MILL 100, IKA JAPAN K.K.).
2. With respect to 5 g of the pulverized pig bone, any of pretreatments (1) through (5) below was carried out.
   (1) No treatment
   (2) Immersion in 30 mL of nitric acid (1 mol/L) at 50°C for 1 day
   (3) Irradiation with microwaves of 500 W for 30 seconds
   (4) Irradiation with microwaves of 500 W for 60 seconds
   (5) Irradiation with microwaves of 500 W for 120 seconds
3. To each of the pig bones of (1) and (3) through (5), 30 mL of nitric acid (1 mol/L) was added. The pig bones of (1) through (5) were immersed in 30 mL of nitric acid at 20°C for 48 hours while being shaken (100 rpm). Thus, the bone tissue was decalcified.
4. A supernatant was collected, and thus an acid extract was obtained.
5. A phosphoric acid concentration was measured with Malachite Green Phosphate Assay Kit (BioAssay Systems). The measurement was carried out in accordance with a manual attached to the product. In the measurement, the supernatant was diluted with distilled water.

### [Results]

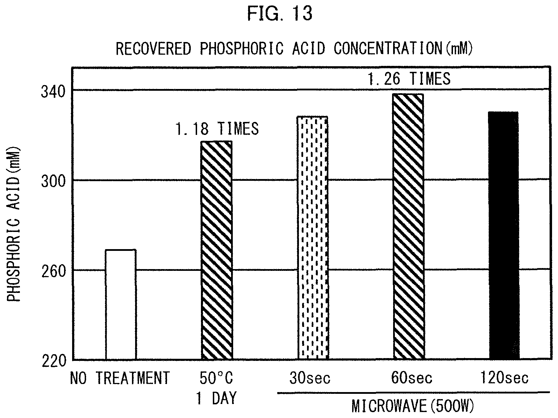

Results are shown in Fig. 13. From the bone tissue (2) pretreated by heating at 50°C, phosphoric acid was extracted in an amount which was 1.18 times that from the bone tissue (1) that was not pretreated. From the bone tissues (3) through (5) which were pretreated by irradiation with microwaves, phosphoric acid was extracted in an amount which was up to 1.26 times that from the bone tissue (1) that was not pretreated.

It has been thus found that an amount of phosphoric acid extracted from an acid extract increases by carrying out appropriate pretreatment. In particular, irradiation with microwaves is a preferable aspect because the treatment is completed in a short time, heating is not necessary, the treatment can be applied to a large bone tissue, and it is possible to further improve a phosphoric acid extraction ratio.

### [Example 5: Analysis of protein component contained in acid extract]

By the following procedure, a protein component contained in an acid extract was analyzed.
1. A pig bone obtained from a slaughterhouse was finely pulverized with a mill (IKA TUBE MILL 100, IKA JAPAN K.K.).
2. To a pig bone in a wet weight of 2 g, 30 mL of hydrochloric acid (1 mol/L) was added, and the pig bone was immersed in the hydrochloric acid at 20°C for 48 hours while being shaken (100 rpm). Thus, the bone tissue was decalcified.
4. A supernatant was collected, and thus an acid extract was obtained.
5. A resultant acid extract was made into (1) an undiluted acid extract solution, (2) a 1/2-diluted acid extract solution, and (3) a 1/4-diluted acid extract solution.
6. A proper amount of 1 mol/L NaOH solution was added to make a pH neutral.
5. An equivalent amount of dithiothreitol was added as a reducing agent, and heating at 95°C was carried out for 10 minutes.
6. With use of a 5% polyacrylamide gel (for high-molecular component) and a 12.5% polyacrylamide gel (for low-molecular component), electrophoresis was carried out according to a common procedure.

### [Results]

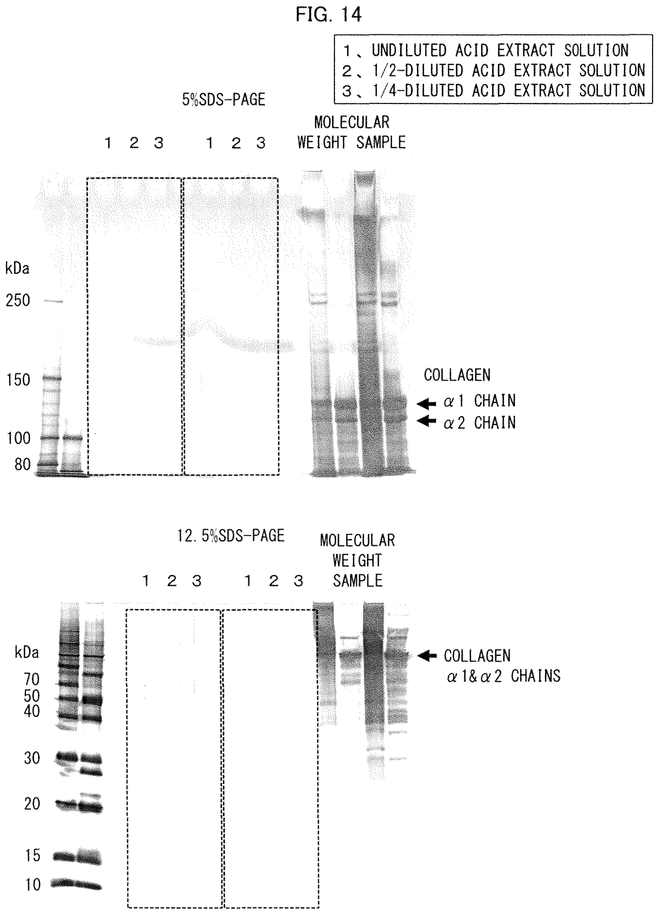

Results are shown in Fig. 14. As control samples, type I collagen, which is a protein contained in a bone in a large amount, and a molecular weight marker were electrophoresed in the same gel. As seen in Fig. 14, a protein having a molecular weight of 10,000 Da or more, which would be stained with a highly sensitive silver staining kit, was not detected from the acid extract. That is, it has been found that the acid extract hardly contains a protein component. This seems to be because the treatment was carried out with hydrochloric acid, which is a strong acid, and therefore a peptide bond of a protein was cleaved to be low-molecular peptide and amino acid.

### [Example 6: Preparation of protease treatment liquid and protein component analysis]

### [Example 6-1: Preparation of protease treatment liquid]

A protease treatment liquid was obtained with a procedure below.
1. Bone tissue was treated with an acid by being immersed in a hydrochloric acid aqueous solution of 1 mol/L for 48 hours.
2. A resultant treatment liquid was sorted out into an acid extract and a bone tissue residue, and the acid extract was transferred to another container.
3. To the bone tissue residue, a citrate buffer solution (pH: 3.5) of 0.1 mol/L was added. The added amount was 10 mL per gram in terms of initial bone weight.
4. The resultant bone tissue residue was immersed in a treatment liquid containing three types of proteases below. Conditions of protease treatment were as follows. Protease concentration: 2% (w/w), temperature: 50°C, pH: optimum pH.
   - Proleather FG-F (Amano Enzyme Inc., derived from Bacillus sp.)
   - Protease P "Amano" 3G (Amano Enzyme Inc., derived from Aspergillus melleus)
   - Protease M "Amano" SD (Amano Enzyme Inc., derived from Aspergillus oryzae)

### [Results]

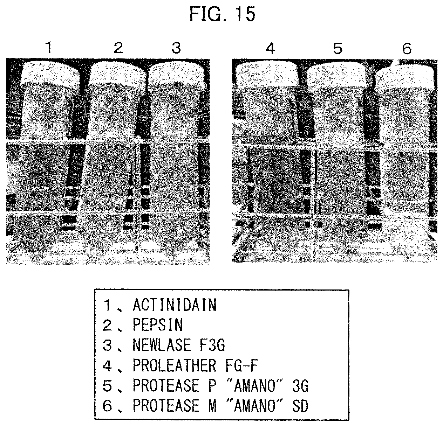

Appearances of the treatment liquids are shown in Fig. 15. As a reference, results of Example 1-5 (Newlase F3G and pepsin) and Example 1-6 (actinidain) are also shown together. As seen in Fig. 15, it has been found that, even in the protease studied in this Example, the bone degradation residue was degraded, and thus a protease treatment liquid could be obtained. Note that a precipitate was seen in the protease treatment liquid obtained in this Example. This seems to be a precipitate of a calcium salt which was generated because the liquid nature was close to neutral. While taking into consideration a result of Example 6-2 described below, it can be said that a protein contained in the bone degradation residue was sufficiently degraded by the treatment with a protease.

### [Example 6-2: Analysis of protein component contained in protease treatment liquid]

In a manner similar to Examples 1-5, 1-6, and 6-1, protease treatment liquids were prepared using proteases below.
- Pepsin (Sigma Aldrich, aspartic protease)
- Peptidase R (Amano Enzyme Inc., derived from Rhizopus)
- Protease P "Amano" 3G (Amano Enzyme Inc., derived from Aspergillus melleus)
- Proleather FG-F (Amano Enzyme Inc., derived from Bacillus sp.)
- Newlase F3G (Amano Enzyme Inc., derived from Rhizopus niveus)
- Protease M "Amano" SD (Amano Enzyme Inc., derived from Aspergillus oryzae)
- Actinidain (cysteine protease derived from a kiwi fruit)

Resultant protease treatment liquids were each diluted 10 times (lane A) or diluted 5 times (lane B), and electrophoresed using a 16% polyacrylamide gel. A protein after the electrophoresis was silver-stained. As a control sample, a solution containing only an enzyme (lane C) was electrophoresed.

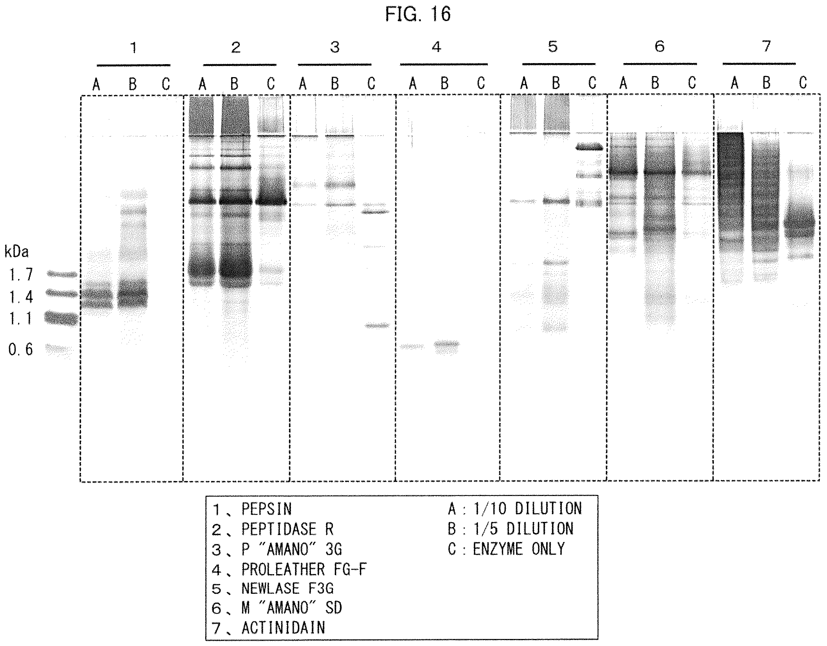

Results are shown in Fig. 16. As illustrated in Fig. 16, all of the protease treatment liquids each contained a protein component having a molecular weight different from that of the enzyme (lane C). That is, even after treatment with any of the proteases, the protein contained in the bone tissue residue was degraded. The peptide thus degraded is used by a plant body as a source of nutrition.

### [Example 7: Recovery of phosphoric acid from bone tissue]

By the following procedure, phosphoric acid was recovered as a precipitate from an acid extract. Moreover, a recovery rate of phosphoric acid was calculated.
1. A pig bone obtained from a slaughterhouse was finely pulverized with a mill (IKA TUBE MILL 100, IKA JAPAN K.K.).
2. The pulverized pig bone was immersed in a nitric acid aqueous solution of 1N or a hydrochloric acid aqueous solution of 1N for 48 hours.
3. A supernatant was collected, and thus an acid extract was obtained.
4. To 0.5 mL of the acid extract, a sodium hydroxide aqueous solution of 5N was added by 30 µL, 50 µL, 70 µL, 100 µL, or 120 µL, and left to stand still at room temperature for 1 hour. Thus phosphoric acid was precipitated.
5. By centrifugal separation, the reaction liquid was divided into a supernatant and a precipitate.
6. To the precipitate, 0.5 mL of a hydrochloric acid aqueous solution of 1N was added, and phosphoric acid was redissolved.
7. Contained amounts of phosphoric acid in the redissolved liquid of the precipitate obtained in the step 5 and in the supernatant obtained in the step 4 were measured. In the measurement, Malachite Green Phosphate Assay Kit (BioAssay Systems) was used. Moreover, a phosphoric acid recovery rate was calculated with respect to a contained amount of phosphoric acid in the acid extract obtained in the step 3.

### [Table 8]

**Table 8**

| Nitric acid aqueous solution | | | | | | |
|---|---|---|---|---|---|---|
| Added amount of NaOH (µL) | Precipitate | | Supernatant | | Precipitate+ supernatant | |
| | Weight (mg) | Recovery rate (%) | Weight (mg) | Recovery rate (%) | Weight (mg) | Recovery rate (%) |
| 50 | 8.86 | 36.0 | 12.07 | 49.0 | 20.9 | 84.9 |
| 70 | 12.8 | 52.0 | 7.93 | 32.2 | 20.7 | 84.1 |
| 100 | 18.5 | 75.2 | 3.36 | 13.6 | 21.9 | 88.8 |
| 120 | 20.5 | 83.4 | 2.47 | 10.0 | 23.0 | 93.4 |

| Hydrochloric acid aqueous solution | | | | | | |
|---|---|---|---|---|---|---|
| Added amount of NaOH (µL) | Precipitate | | Supernatant | | Precipitate+ supernatant | |
| | Weight (mg) | Recovery rate (%) | Weight (mg) | Recovery rate (%) | Weight (mg) | Recovery rate (%) |
| 30 | 6.48 | 41.7 | 8.30 | 54.9 | 14.8 | 97.7 |
| 50 | 8.18 | 52.7 | 7.78 | 51.4 | 16.0 | 106 |
| 70 | 13.6 | 72.5 | 1.52 | 25.7 | 15.1 | 100 |
| 100 | 15.1 | 97.5 | 1.87 | 12.3 | 17.0 | 112 |

### [Results]

Results are shown in Table 8. For the acid extract using nitric acid, 70% or more of phosphoric acid could be recovered as a precipitate when the added amount of the NaOH aqueous solution was 100 pL or more. Meanwhile, phosphoric acid contained in the supernatant was decreased to approximately 10% when the added amount of the NaOH aqueous solution was 120 µL. For the acid extract using hydrochloric acid, 70% or more of phosphoric acid could be recovered as a precipitate when the added amount of the NaOH aqueous solution was 70 µL or more. Meanwhile, phosphoric acid contained in the supernatant was decreased to approximately 10% when the added amount of the NaOH aqueous solution was 100 µL. Thus, by adding a proper amount of the NaOH aqueous solution to the acid extract, it was possible to recover and roughly purify phosphoric acid as a precipitate.

A contained amount of phosphoric acid in the acid extract using nitric acid was 24.6 mg. This amount is equivalent to 34.7% with respect to the wet weight (71 mg) of the pig bone used in the step 2. A contained amount of phosphoric acid in the acid extract using hydrochloric acid was 15.1 mg. This amount is equivalent to 20.2% with respect to the wet weight (74.5 mg) of the pig bone used in the step 2. A weight of phosphoric acid accounting for bone tissue before drying is assumed to be 25% to 35%. Therefore, by preparation of an acid extract, it is possible to take out phosphoric acid by up to 99% (= 34.7/35 × 100).

### [Example 8: Purification of phosphoric acid by adding sulfate]

### [Example 8-1: Study of phosphoric acid recovery rate from supernatant]

From the redissolved liquid of the phosphoric acid precipitate obtained in Example 7, calcium was removed as a calcium sulfate precipitate by the following procedure.
1. In a manner similar to Example 7, a redissolved liquid of a phosphoric acid precipitate was prepared. An acid used in the step 2 of Example 7 was a nitric acid aqueous solution of 1N or a hydrochloric acid aqueous solution of 1N. An amount of a sodium hydroxide aqueous solution of 5N added in the step 4 of Example 7 was 100 µL.
2. Sulfuric acid or sulfate (sodium sulfate, potassium sulfate, ammonium sulfate, or magnesium sulfate) was added. An added amount of the sulfuric acid or sulfate was set so that a final concentration was 0.4 M, 0.6 M, 0.8 M, or 1.0 M.
3. The generated calcium sulfate precipitate was removed by centrifugal separation.
4. A weight of phosphoric acid contained in the supernatant was measured. In the measurement, Malachite Green Phosphate Assay Kit (BioAssay Systems) was used. A phosphoric acid recovery rate was calculated with respect to a contained amount of phosphoric acid in the precipitate (the precipitate obtained in the step 5 of Example 7) which was generated by adding the sodium hydroxide aqueous solution to the acid extract before adding the sulfuric acid
or sulfate.

### [Table 9]

**Table 9**

| Nitric acid aqueous solution | | | | | | | | | | |
|---|---|---|---|---|---|---|---|---|---|---|
| Concentration (M) | H₂SO₄ | | Na₂SO₄ | | K₂SO₄ | | (NH₄)₂SO₄ | | MgSO₄ | |
| | Weight (mg) | Recovery rate (%) | Weight (mg) | Recovery rate (%) | Weight (mg) | Recovery rate (%) | Weight (mg) | Recovery rate (%) | Weight (mg) | Recovery rate (%) |
| 0.4 | 11.6 | 62.6 | 12.6 | 68.1 | 12.7 | 68.7 | 11.0 | 59.6 | 10.9 | 58.8 |
| 0.6 | 12.4 | 66.7 | 11.4 | 61.5 | 9.82 | 53.0 | 11.6 | 62.7 | 11.9 | 64.2 |
| 0.8 | 13.2 | 71.5 | 11.0 | 59.3 | 9.69 | 52.3 | 11.5 | 62.3 | 11.4 | 61.6 |
| 1.0 | 12.3 | 66.5 | 11.2 | 60.5 | 9.47 | 51.1 | 11.7 | 63.2 | 11.5 | 62.2 |
| Phosphoric acid concentration at highest recovery rate | 169mM (0.8mL) | | 257mM (0.5mL) | | 260mM (0.5mL) | | 239mM (0.5mL) | | 243mM (0.5mL) | |

| Hydrochloric acid aqueous solution | | | | | | | | | | |
|---|---|---|---|---|---|---|---|---|---|---|
| Concentration (M) | H₂SO₄ | | Na₂SO₄ | | K₂SO₄ | | (NH₄)₂SO₄ | | MgSO₄ | |
| | Weight (mg) | Recovery rate (%) | Weight (mg) | Recovery rate (%) | Weight (mg) | Recovery rate (%) | Weight (mg) | Recovery rate (%) | Weight (mg) | Recovery rate (%) |
| 0.4 | 11.5 | 84.4 | 11.9 | 87.5 | 12.7 | 93.4 | 13.1 | 96.7 | 12.5 | 91.8 |
| 0.6 | 12.8 | 94.5 | 12.0 | 88.5 | 10.1 | 74.7 | 12.6 | 92.6 | 12.3 | 90.2 |
| 0.8 | 13.4 | 98.8 | 12.3 | 90.5 | 11.6 | 85.3 | 12.2 | 90.0 | 11.7 | 86.3 |
| 1.0 | 13.1 | 96.3 | 11.9 | 87.5 | 11.3 | 83.1 | 12.4 | 91.2 | 11.8 | 86.7 |
| Phosphoric acid concentration at highest recovery rate | 171mM (0.8mL) | | 251mM (0.5mL) | | 259mM (0.5mL) | | 267mM (0.5mL) | | 255mM (0.5mL) | |

### [Results]

Results are shown in Table 9. It has been found that, by appropriately setting a sulfate concentration, a phosphoric acid recovery rate equivalent to that by sulfuric acid can be achieved even by sulfate. At a low concentration of 0.4 M, the phosphoric acid concentration obtained by adding the sulfate tended to be slightly higher than the phosphoric acid concentration obtained by adding the sulfuric acid. Importantly, addition of the sulfate not only can reduce a volume of the system as compared with addition of the sulfuric acid, but also can achieve a resultant phosphoric acid concentration which is approximately 1.5 times higher. In view of this, the method in accordance with an embodiment of the present invention is useful in purification of phosphoric acid. Moreover, in practical use of the technique, an added amount of sulfuric acid or sulfate is preferably lower. From this fact also, efficacy of the calcium removal effect by sulfate is proved.

Using sulfate instead of sulfuric acid in precipitation of calcium brings about the following advantages.
- A phosphoric acid concentration is approximately 1.5 times higher.
- An increase in volume of the reaction system can be suppressed because a liquid is not added.
- The reaction system does not become strongly acidic and therefore an amount of a base necessary for neutralization can be small.
- Highly safe.

### [Example 8-2: Study of calcium removal ability from supernatant)

A contained amount of calcium in the supernatant obtained in the step 4 of Example 8-1 was measured. In the measurement, LAQUAtwin-Ca-11 was used. A remaining percentage of calcium was calculated with respect to a contained amount of calcium in the precipitate (the precipitate obtained in the step 5 of Example 7) which was generated by adding the sodium hydroxide aqueous solution to the acid extract before adding the sulfuric acid or sulfate.

### [Table 10]

**Table 10**

| Nitric acid aqueous solution | | | | | | | | | | |
|---|---|---|---|---|---|---|---|---|---|---|
| Concentration (M) | H₂SO₄ | | Na₂SO₄ | | K₂SO₄ | | (NH₄)₂SO₄ | | MgSO₄ | |
| | Weight (*µ* g) | Retention (%) | Weight (*µ* g) | Retention (%) | Weight (*µ* g) | Retention (%) | Weight (*µ* g) | Retention (%) | Weight (*µ* g) | Retention (%) |
| 0.4 | 2200 | 27.6 | 320 | 4.01 | 435 | 5.45 | 387 | 4.85 | 700 | 8.77 |
| 0.6 | 975 | 12.2 | 170 | 2.13 | 312 | 3.91 | 165 | 2.07 | 295 | 3.70 |
| 0.8 | 600 | 7.52 | 115 | 1.44 | 216 | 2.71 | 115 | 1.44 | 175 | 2.19 |
| 1.0 | 432 | 5.41 | 90.0 | 1.13 | 28.0 | 0.351 | 95.0 | 1.19 | 120 | 1.50 |
| Ca²⁺ concentration at lowest retention | 12mM (0.9mL) | | 4.5mM (0.5mL) | | 1.8mM (0.4mL) | | 4.8mM (0.5mL) | | 6.0mM (0.5mL) | |

| Hydrochloric acid aqueous solution | | | | | | | | | | |
|---|---|---|---|---|---|---|---|---|---|---|
| Concentration (M) | H₂SO₄ | | Na₂SO₄ | | K₂SO₄ | | (NH₄)₂SO₄ | | MgSO₄ | |
| | Weight (*µ* g) | Retention (%) | Weight (*µ* g) | Retention (%) | Weight (*µ* g) | Retention (%) | Weight (*µ* g) | Retention (%) | Weight (*µ* g) | Retention (%) |
| 0.4 | 1210 | 18.3 | 155 | 2.35 | 215 | 3.26 | 155 | 2.35 | 270 | 4.09 |
| 0.6 | 650 | 9.85 | 90.0 | 1.36 | 172 | 2.61 | 80.0 | 1.21 | 125 | 1.89 |
| 0.8 | 512 | 7.76 | 65.0 | 0.985 | 31. 5 | 0.477 | 70.0 | 1.06 | 95.0 | 1.44 |
| 1.0 | 297 | 4.50 | 50.0 | 0.758 | <18.0 | <0.273 | 50.0 | 0.758 | 75.0 | 1.14 |
| Ca2+ concentration at lowest retention | 8.3mM (0.9mL) | | 2.5mM (0.5mL) | | <1.0mM (0.5mL) | | 2.5mM (0.5mL) | | 3.8mM (0.5mL) | |

### [Results]

Results are shown in Table 10. In general, sulfate had a lower remaining percentage of calcium than sulfuric acid at the same concentration. For example, in the experimental system in which 0.4 M sodium hydroxide was added after treatment with hydrochloric acid, a calcium amount in the solution was 1,210 µg with H₂SO₄, whereas 155 µg to 270 µg with sulfate. That is, the calcium removal ability in this condition was up to 7.8 times higher with sulfate. In addition, importantly, when comparing the solution containing sulfate with the solution containing sulfuric acid, the volume of the former solution was approximately 1/2 of the latter while the calcium concentration of the former was decreased to approximately 1/2 of the latter. That is, by adding sulfate, it is possible to efficiently remove calcium which is unnecessary in purification of phosphoric acid.

In comparison in terms of the acid used in acid treatment, a remaining percentage of calcium was lower with acid treatment by hydrochloric acid than with acid treatment by nitric acid. Calcium was mostly removed in the system in which 1 M of potassium sulfate was added to the acid extract which had been obtained by treatment with hydrochloric acid.

Table 11 shows a value of a relative ratio (%) of calcium amount (mg)/phosphoric acid amount (mg) calculated based on the results of Tables 9 and 10. Although phosphoric acid can be recovered by adding sulfuric acid, it has been shown that, at any concentration, a residual amount of calcium was larger than that in the experimental system in which sulfate was added. Meanwhile, when phosphoric acid was recovered by adding sulfate, a residual amount of calcium was remarkably smaller than that in the experimental system in which sulfuric acid was added. Thus, a relatively larger recovery amount of phosphoric acid was seen. Thus, by adding appropriate sulfate, a remaining amount of calcium can be reduced to 0.2% by weight without reducing a recovery amount of phosphoric acid. Therefore, it can be said that, in purification of phosphoric acid extracted from bone tissue, it is preferable to use sulfate rather than sulfuric acid.

### [Table 11]

**Table 11**

| Nitric acid aqueous solution | | | | | |
|---|---|---|---|---|---|
| Concentration | Calcium (mg) /Phosphoric acid (mg) × 100 (%) | | | | |
| (M) | H₂SO₄ | Na₂SO₄ | K₂SO₄ | (NH₄)₂SO₄ | MgSO₄ |
| 0.4 | 19.0 | 2.5 | 3.4 | 3.5 | 6.4 |
| 0.6 | 7.9 | 1.5 | 3.2 | 1.4 | 2.5 |
| 0.8 | 4.5 | 1.1 | 2.2 | 1.0 | 1.5 |
| 1.0 | 3.5 | 0.8 | 0.3 | 0.8 | 1.0 |

| Hydrochloric acid aqueous solution | | | | | |
|---|---|---|---|---|---|
| Concentration | Calcium (mg) /Phosphoric acid (mg) × 100 (%) | | | | |
| (M) | H₂SO₄ | Na₂SO₄ | K₂SO₄ | (NH₄)₂SO₄ | MgSO₄ |
| 0.4 | 10.6 | 1.3 | 1.7 | 1.2 | 2.2 |
| 0.6 | 5.1 | 0.7 | 1.7 | 0.6 | 1.0 |
| 0.8 | 3.8 | 0.5 | 0.3 | 0.6 | 0.8 |
| 1.0 | 2.3 | 0.4 | 0.2 | 0.4 | 0.6 |

### [Example 8-3: Study of ratio of phosphoric acid which leaks to precipitate]

A contained amount of phosphoric acid in the calcium sulfate precipitate obtained in the step 3 of Example 8-1 was measured with a procedure below.
1. To the calcium sulfate precipitate, EDTA (pH: 7.4) and sodium hydroxide of 5N were added, and the precipitate was redissolved.
2. A contained amount of phosphoric acid in the obtained solution was measured. In the measurement, Malachite Green Phosphate Assay Kit (BioAssay Systems) was used. A phosphoric acid leakage ratio was calculated with respect to a contained amount of phosphoric acid in the precipitate (the precipitate obtained in the step 5 of Example 7) which was generated by adding the sodium hydroxide aqueous solution to the acid extract before adding the sulfuric acid or sulfate.

### [Table 12]

**Table 12**

| Nitric acid aqueous solution | | | | | | | | | | |
|---|---|---|---|---|---|---|---|---|---|---|
| Concentration (M) | H₂SO₄ | | Na₂SO₄ | | K₂SO₄ | | (NH₄)₂SO₄ | | MgSO₄ | |
| | Weight (mg) | Leakage ratio (%) | Weight (mg) | Leakage ratio (%) | Weight (mg) | Leakage ratio (%) | Weight (mg) | Leakage ratio (%) | Weight (mg) | Leakage ratio (%) |
| 0.4 | 1.53 | 8.23 | 2.00 | 10.8 | 2.15 | 11.6 | 2.81 | 15.1 | 2.45 | 13.2 |
| 0.6 | 1.97 | 10.6 | 1.69 | 9.13 | 5.29 | 28.5 | 2.38 | 12.9 | 4.32 | 23.3 |
| 0.8 | 1.43 | 7.70 | 1.71 | 9.25 | 5.22 | 28.2 | 1.54 | 8.32 | 1.96 | 10.6 |
| 1.0 | 2.01 | 10.8 | 1.74 | 9.41 | 4.80 | 25.9 | 1.50 | 8.11 | 1.84 | 9.93 |

| Hydrochloric acid aqueous solution | | | | | | | | | | |
|---|---|---|---|---|---|---|---|---|---|---|
| Concentration (M) | H₂SO₄ | | Na₂SO₄ | | K₂SO₄ | | (NH₄)₂SO₄ | | MgSO₄ | |
| | Weight (mg) | Leakage ratio (%) | Weight (mg) | Leakage ratio (%) | Weight (mg) | Leakage ratio (%) | Weight (mg) | Leakage ratio (%) | Weight (mg) | Leakage ratio (%) |
| 0.4 | 1.18 | 8.67 | 0.776 | 5.71 | 0.957 | 7.05 | 1.2 | 8.87 | 1.36 | 10.1 |
| 0.6 | 0.923 | 6.80 | 0.716 | 5.27 | 3.73 | 27.5 | 2.25 | 16.6 | 1.67 | 12.3 |
| 0.8 | 0.824 | 6.07 | 0.705 | 5.19 | 2.37 | 17.5 | 1.49 | 10.9 | 1.55 | 11.4 |
| 1.0 | 0.782 | 5.76 | 0.705 | 5.19 | 2.45 | 18.1 | 1.13 | 8.34 | 1.49 | 11.0 |

### [Results]

Results are shown in Table 12. As seen in Table, by adjusting the sulfate concentration as appropiate, a contained amount of phosphoric acid in the calcium sulfate precipitate could be reduced to an amount equivalent to that by sulfuric acid. A sulfate concentration of 0.4 M was able to sufficiently reduce phosphoric acid to leak.

### [Example 8-4: Study of total phosphoric acid recovery rate]

A total amount of the phosphoric acid recovery rate from the supernatant studied in Example 8-1 and the ratio of phosphoric acid which leaks to the precipitate studied in Example 8-3 was studied. There is prospect that it is possible to recover phosphoric acid contained in a precipitate again by cleaning the precipitate. For example, by cleaning a precipitate (calcium sulfate) with pure water, it is possible to recover phosphoric acid contained in the precipitate. Therefore, as the total contained amount of phosphoric acid increases, a potential amount of phosphoric acid that can be recovered increases.

### [Table 13]

**Table 13**

| Nitric acid aqueous solution | | | | | | | | | | |
|---|---|---|---|---|---|---|---|---|---|---|
| Concentration (M) | H₂SO₄ | | Na₂SO₄ | | K₂SO₄ | | (NH₄)₂SO₄ | | MgSO₄ | |
| | Weight (mg) | Recovery rate (%) | Weight (mg) | Recovery rate (%) | Weight (mg) | Recovery rate (%) | Weight (mg) | Recovery rate (%) | Weight (mg) | Recovery rate (%) |
| 0.4 | 13.1 | 70.9 | 14.6 | 78.9 | 14.9 | 80.3 | 13.9 | 74.8 | 13.3 | 72.0 |
| 0.6 | 14.3 | 77.3 | 13.1 | 70.6 | 15.1 | 81.5 | 14.0 | 75.5 | 16.2 | 87.5 |
| 0.8 | 14.7 | 79.2 | 12.7 | 68.5 | 14.9 | 80.5 | 13.1 | 70.6 | 13.4 | 72.1 |
| 1.0 | 14.3 | 77.4 | 13.0 | 69.9 | 14.3 | 77.0 | 13.2 | 71.3 | 13.4 | 72.1 |

| Hydrochloric acid aqueous solution | | | | | | | | | | |
|---|---|---|---|---|---|---|---|---|---|---|
| Concentration (M) | H₂SO₄ | | Na₂SO₄ | | K₂SO₄ | | (NH₄)₂SO₄ | | MgSO₄ | |
| | Weight (mg) | Recovery rate (%) | Weight (mg) | Recovery rate (%) | Weight (mg) | Recovery rate (%) | Weight (mg) | Recovery rate (%) | Weight (mg) | Recovery rate (%) |
| 0.4 | 12.6 | 93. 1 | 12.7 | 93.2 | 13.6 | 100 | 14.3 | 106 | 13.8 | 102 |
| 0. 6 | 13.7 | 101 | 12.7 | 93. 8 | 13.9 | 102 | 14.8 | 109 | 13.9 | 103 |
| 0.8 | 14.2 | 105 | 13.0 | 95. 7 | 13.9 | 103 | 13. 7 | 101 | 13.3 | 97.8 |
| 1.0 | 13.9 | 102 | 12.6 | 92. 7 | 13.7 | 101 | 13.5 | 100 | 13.3 | 97. 7 |

### [Results]

Results are shown in Table 13. It has been found that, by appropiately adjusting the concentration, a phosphoric acid recovery rate equivalent to that by sulfuric acid can be achieved even by sulfate. A total recovery rate was higher for the acid extract using hydrochloric acid. An absolute contained amount of phosphoric acid was similar between the acid extract using hydrochloric acid and the acid extract using nitric acid.

### [Example 9: RNA expression analysis 2 of plant body]

### [Preparation of cDNA library and gene expression analysis by next-generation sequencer]

A procedure similar to that of Example 3 was carried out.

### [Analysis of gene whose expression amount was changed]

A procedure similar to that of Example 3 was carried out. Comparison of expression amounts was carried out with two sets, i.e., (i) a bone solubilization liquid B vs. a commercially available liquid fertilizer, and (ii) a mixed solution of a bone solubilization liquid B and a commercially available liquid fertilizer vs. a commercially available liquid fertilizer only.

### [Results]

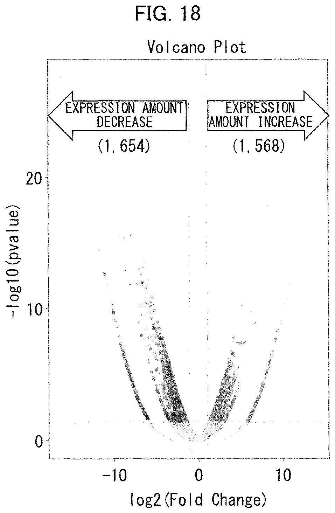

Fig. 18 shows a result of analyzing an expression variable gene in a leaf of a sprout which was cultivated for 5 days while providing a bone solubilization liquid B or a commercially available liquid fertilizer. The number of genes for which an FPKM value was increased by 2 times or more was 1,568. The number of genes for which an FPKM value was decreased to 1/2 or less was 1,654. As such, the expression amount changed for a total of 3,222 genes. As a result of the GO enrichment analysis, the genes for which the expression amount changed included: 65 genes related to control of growth; 44 genes related to photosynthesis; and 26 genes related to light-harvesting in photosynthetic photosystem I.

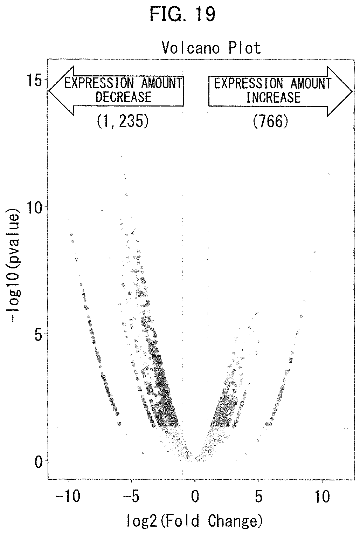

Fig. 19 shows a result of analyzing an expression variable gene in a root of a sprout which was cultivated for 5 days while providing a mixed solution of a bone solubilization liquid B and a commercially available liquid fertilizer or only a commercially available liquid fertilizer. The number of genes for which an FPKM value was increased by 2 times or more was 766. The number of genes for which an FPKM value was decreased to 1/2 or less was 1,235. As such, the expression amount changed for a total of 2,001 genes. As a result of the GO enrichment analysis, the genes for which the expression amount changed included: 17 genes related to nitric acid assimilation; 16 genes related to root hair elongation; and 12 genes related to nitric acid uptake.

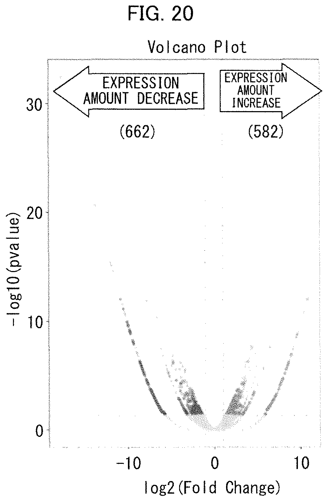

Fig. 20 shows a result of analyzing an expression variable gene in a leaf of a sprout which was cultivated for 5 days while providing a mixed solution of a bone solubilization liquid B and a commercially available liquid fertilizer or only a commercially available liquid fertilizer. The number of genes for which an FPKM value was increased by 2 times or more was 582. The number of genes for which an FPKM value was decreased to 1/2 or less was 662. As such, the expression amount changed for a total of 1,244 genes. As a result of the GO enrichment analysis, the genes for which the expression amount changed included: 32 genes related to a response to abscisic acid (plant hormone); 13 genes related to root hair elongation; and six genes related to promotion of germination.

From the results shown in Figs. 18 through 20, it has been found that the bone solubilization liquid B alters gene expression of the sprout. In particular, expression of gene groups related to tissue growth in the leaf or root portion, photosynthesis in the leaf, and nutrient absorption in the root highly varied.

### [Example 10: Study of calcium ion removal efficiency by addition of sulfate]

By adding sulfate to a redissolved liquid of a phosphoric acid precipitate, calcium ions were removed as a calcium carbonate precipitate. A contained amount of calcium ions in a supernatant after removal of the precipitate was measured, and thus calcium removal efficiency was studied. A specific procedure was as follows.
1. In a manner similar to Example 7, a redissolved liquid of a phosphoric acid precipitate was prepared. An acid used in the step 2 of Example 7 was a hydrochloric acid aqueous solution of 1N. An amount of a sodium hydroxide aqueous solution of 5N added in the step 4 of Example 7 was 100 µL.
2. Liquid sulfuric acid (H₂SO₄) or solid sulfate (Na₂SO₄, K₂SO₄, Mg₂SO₄, and (NH₄)₂SO₄) was added. An added amount of the sulfuric acid or sulfate was set so that a final concentration was 0.4 M.
3. The generated calcium sulfate precipitate was removed by centrifugal separation.
4. A weight (mg) of calcium ions contained in the supernatant was measured. Measurement of calcium ions was carried out under a precise measurement condition using high-speed ion chromatography IC-8100EX (Tosoh Corporation) connected with TSKgel SuperIC-Cation HSII (4.6 mm I.D. × 10 cm). A mixed solution containing 3.0 mmol/L of methanesulfonic acid and 2.7 mmol/L of 18-crown-6 was used as an eluting solution. A measurement temperature was 40°C, a flow velocity was 1.0 mL/min, and an injection amount was 30 µL. An electric conductivity (µS) was measured, and a regression formula was obtained from an area of a reference material. Thus, a calcium ion concentration was measured. From the calcium ion concentration, a contained amount of calcium ions per sample (30 uL) was obtained.

### [Table 14]

**Table 14**

| | Before addition | After addition | | | | |
|---|---|---|---|---|---|---|
| | | H₂SO₄ | Na₂SO₄ | K₂SO₄ | (NH₄)₂SO₄ | MgSO₄ |
| Ca²⁺ content (mg) | 5.24 | 1. 41 | 0. 327 | 0. 345 | 0. 397 | 0. 461 |
| Removal rate (%) | - | 73 | 94 | 93 | 92 | 91 |

### [Results]

Results are shown in Table 14. In the system in which the liquid sulfuric acid was added, a capacity of the whole system increased, and a removal rate of calcium ions was lowest, i.e., 73%. In the system in which the solid sulfate was added, an increase in capacity of the whole system was small, and a removal rate of calcium ions was also high, i.e., 91% to 94%. Moreover, the method in which sulfate is added is highly safe because sulfuric acid, which is a strong acid, is not used. Moreover, by increasing or decreasing the added amount of sulfate as appropriate, it was possible to cause an intended concentration of calcium to remain.

In producing phosphoric acid or a fertilizer containing phosphoric acid from bone tissue, calcium ions contained in the bone tissue causes reduction of recovery of phosphoric acid. This is because, in the neutral zone, calcium ions bind to phosphate ions to form a calcium phosphate precipitate. Therefore, it has been shown that it is preferable to remove calcium ions by precipitation, and calcium ions can be removed successfully by adding sulfate.

Potassium ions, magnesium ions, or ammonium ions contained in sulfate are components necessary for plant growth. Therefore, in a case where potassium sulfate, magnesium sulfate, or ammonium sulfate is used as sulfate, a component necessary for plant growth can be contained in a fertilizer. Alternatively, even in a case where the base added in the step 4 of Example 7 is changed from sodium hydroxide to potassium hydroxide, a component necessary for plant growth can be contained in a fertilizer. By employing such a production method, it is possible to produce a fertilizer having an increased value.

### [Example 11: Purification of phosphoric acid by addition of carbonate]

From the redissolved liquid of the phosphoric acid precipitate obtained in Example 7, calcium was removed as a calcium carbonate precipitate by the following procedure.
1. In a manner similar to Example 7, a redissolved liquid of a phosphoric acid precipitate was prepared. An acid used in the step 1 of Example 7 was a nitric acid aqueous solution of 1N or a hydrochloric acid aqueous solution of 1N. An amount of a sodium hydroxide aqueous solution of 5N added in the step 4 of Example 7 was 100 µL.
2. Carbonate (solid sodium hydrogen carbonate or sodium carbonate) was added. An added amount of sodium hydrogen carbonate was set so that a final concentration was 0.4 M. An added amount of sodium carbonate was set so that a final concentration was 0.6 M.
3. A generated calcium carbonate precipitate was removed by centrifugal separation.
4. A concentration (ppm) of phosphate ions contained in the supernatant was measured. Measurement of phosphate ions was carried out under a precise measurement condition using high-speed ion chromatography IC-8100EX (Tosoh Corporation) connected with TSKgel SuperIC-Anion HS (4.6 mm I.D. × 10 cm). A mixed solution containing 7.5 mmol/L of sodium hydrogen carbonate and 0.8 mmol/L of sodium carbonate was used as an eluting solution. A measurement temperature was 40°C, a flow velocity was 1.5 mL/min, and an injection amount was 30 µL. An electric conductivity (µS) was measured, and a regression formula was obtained from an area of a reference material. Thus, a phosphate ion concentration was measured. From the phosphate ion concentration, a contained amount of phosphate ions per sample (30 µL) was obtained.

### [Table 15]

**Table 15**

| | Add NaHCO₃ | Add Na₂CO₃ |
|---|---|---|
| | 0.4M | 0.6M |
| Acid extract by hydrochloric acid | 9,800 | 11,400 |
| Acid extract by nitric acid | 1,670 | 2,850 |

### [Results]

Results are shown in Table 15. By adding carbonate, calcium ions precipitated as calcium carbonate, and phosphate ions remained in the supernatant. For example, in the system in which sodium hydrogen carbonate was added to the acid extract by hydrochloric acid, 9,000 ppm or more of phosphoric acid was contained in the supernatant. The carbonate ions remaining in the supernatant can be easily removed because those ions are converted to carbon dioxide by heating.

### [Example 12: Removal of cations contained in sulfate]

### [Example 12-1: Removal of sodium ions or potassium ions]

By the following procedure, cations (sodium ions or potassium ions) contained in sulfate added to remove calcium ions were removed. Specifically, a strong cation exchange gel was used to adsorb sodium ions or potassium ions.
1. TSKgel SP-TOYOPEARL 650 M gel (Tosoh Corporation) was subjected to decantation with pure water, and introduced into a Microspin column (GE Healthcare).
2. A proper amount of a supernatant from which calcium sulfate had been removed by adding sulfate was added to an upper layer of the gel.
3. By carrying out centrifugation by a desktop centrifuge, the gel was caused to pass through the supernatant. Pass-by fractions which passed through the gel were gathered.
3. An amount (mg) of sodium ions or potassium ions contained in the pass-by fractions was measured. In measurement of sodium ions, LAQUAtwin-Na-11 (HORIBA Advanced Techno, Co., Ltd.) was used. In measurement of potassium ions, LAQUAtwin-K-11 (HORIBA Advanced Techno, Co., Ltd.) was used.

### [Table 16]

**Table 16**

| | Sodium ion | Potassium ion |
|---|---|---|
| Undiluted solution(mg) | 11.6 | 11.2 |
| Pass-by fraction(mg) | 1.96 | 1.56 |
| Removal rate (%) | 83.1 | 86.1 |

### [Results]

Results are shown in Table 16. A supernatant obtained after calcium sulfate had been precipitated by adding sodium sulfate or potassium sulfate contained sodium ions or potassium ions. Those ions could be removed by passing the supernatant through the strong cation exchange gel. Specifically, 83% of sodium ions was removed, and 86% of potassium ions was removed. More cations can be removed by repeating the process of passing the supernatant through the strong cation exchange gel.

Thus, it has been shown that, in the method in which sulfate is added, cations added as sulfate can be removed with the strong cation exchange gel. Note, however, that, in a case where a fertilizer is produced from bone tissue, potassium ions, magnesium ions, and ammonium ions do not need to be removed because those ions are important nutrient elements for plant growth.

### [Example 12-2: Removal of magnesium ions or ammonium ions]

By the following procedure, cations (magnesium ions or ammonium ions) contained in sulfate added to remove calcium ions were removed. Specifically, a strong cation exchange gel was used to adsorb magnesium ions or ammonium ions.
1. TSKgel SP-TOYOPEARL 650 M gel (Tosoh Corporation) was subjected to decantation with pure water, and introduced into a Microspin column (GE Healthcare).
2. A proper amount of a supernatant from which calcium sulfate had been removed by adding sulfate was added to an upper layer of the gel.
3. By carrying out centrifugation by a desktop centrifuge, the gel was caused to pass through the supernatant. Pass-by fractions which passed through the gel were gathered.
3. An amount (µg) of magnesium ions, ammonium ions, or calcium ions contained in the pass-by fractions was measured. Measurement of the ion concentration was carried out under a precise measurement condition using high-speed ion chromatography IC-8100EX (Tosoh Corporation) connected with TSKgel SuperIC-Cation HSII (4.6 mm I.D. × 10 cm). A mixed solution containing 3.0 mmol/L of methanesulfonic acid and 2.7 mmol/L of 18-crown-6 was used as an eluting solution. A measurement temperature was 40°C, a flow velocity was 1.0 mL/min, and an injection amount was 30 µL. An electric conductivity (µS) was measured, and a regression formula was obtained from an area of a reference material. Thus, a calcium ion concentration was measured. From the ion concentration, a contained amount of ions per sample (30 µL) was obtained.

### [Table 17]

**Table 17**

| | Add MgSO₄ | | Add (NH₄)₂SO₄ | |
|---|---|---|---|---|
| | Mg²⁺ | Ca²⁺ | NH⁴⁺ | Ca²⁺ |
| Undiluted solution(µg) | 3,770 | 461 | 5,910 | 397 |
| Pass-by fraction (*µ*g) | 19.5 | 14.6 | 827 | 10.8 |
| Removal rate(%) | 99.5 | 96.8 | 86 | 97.3 |

### [Results]

Results are shown in Table 17. It has been shown that magnesium ions, ammonium ions, and calcium ions can also be removed from the supernatant by bringing the supernatant into contact with the strong cation exchange gel. More cations, including calcium ions, can be removed by repeating the process of passing the supernatant through the strong cation exchange gel. Thus, it has been suggested that purity of phosphoric acid can be enhanced.

### [Example 13: Purification of phosphoric acid from commercially available bone meal]

By the following procedure, commercially available steamed bone meal (Omiya Green Service, K.K.) was solubilized to recover phosphoric acid.
1. To 1 g of the steamed bone meal, 10 mL of sulfuric acid (1N), hydrochloric acid (1N), or nitric acid (1N) was added, and a resultant mixture was shaken at 25°C for 19 hours.
2. A resultant acid extract was divided into a supernatant and a precipitate using a centrifuge.
3. To 0.5 mL of the supernatant, 0.1 mL of an NaOH aqueous solution (5N) was added, and a resultant mixture was shaken for 1 hour.
4. By centrifugal separation, a reaction liquid was divided into a supernatant and a precipitate.
5. A proper amount of hydrochloric acid (1N) was added to the precipitate to dissolve the precipitate.
6. The steps 3 through 5 were repeated a plurality of times. Subsequently, the precipitate was dissolved in 0.5 mL of hydrochloric acid (0.4N). Thus, a precipitate solution in which phosphoric acid was concentrated was obtained.
7. An amount of anions contained in the obtained precipitate solution was measured. Measurement was carried out under a precise measurement condition using high-speed ion chromatography IC-8100EX (Tosoh Corporation) connected with TSKgel SuperIC-Anion HS (4.6 mm I.D. × 10 cm). A mixed solution containing 7.5 mmol/L of sodium hydrogen carbonate and 0.8 mmol/L of sodium carbonate was used as an eluting solution. A measurement temperature was 40°C, a flow velocity was 1.5 mL/min, and an injection amount was 30 µL. An electric conductivity (µS) was measured, and a regression formula was obtained from an area of a reference material. Thus, an anion concentration was measured. From the anion concentration, a contained amount of anions per sample (30 µL) was obtained.

### [Table 18]

**Table 18**

| Detected anion | Added acid | | |
|---|---|---|---|
| | Hydrochloric acid | Nitric acid | Sulfuric acid |
| Phosphate ion (ppb) | 17.1 | 18.2 | 5.34 |
| Chloride ion (ppm) | 0 | 0 | 0 |
| Nitrate ion (ppm) | 0 | 0 | 0 |
| Sulfate i on (ppb) | 0 | 0 | 7.14 |

### [Results]

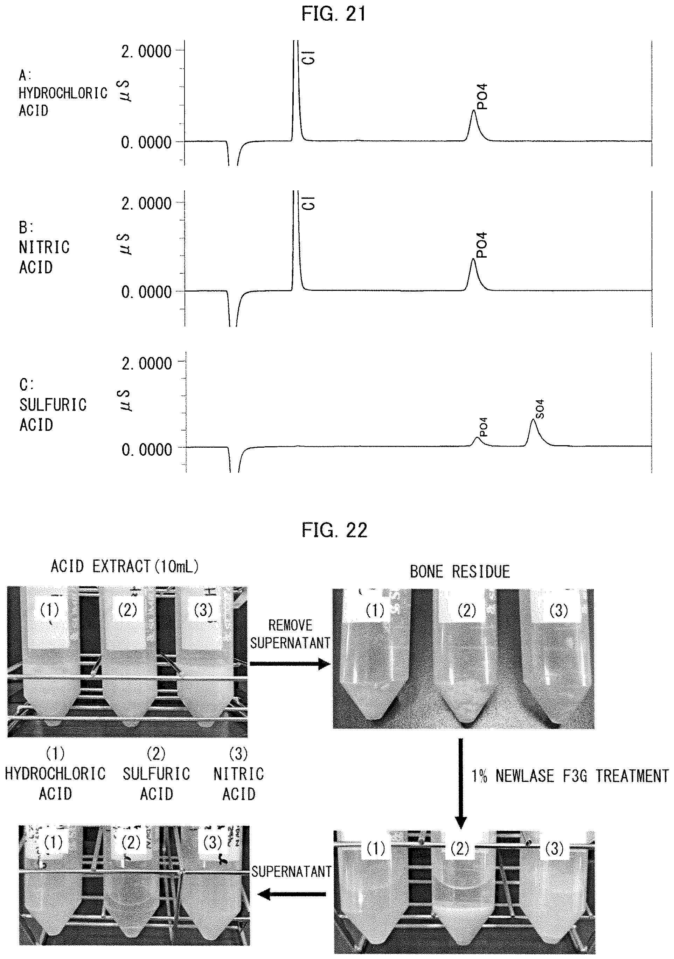

Results are shown in Table 18 and Fig. 21. Even in the case where the commercially available steamed bone meal (bone tissue heated under high pressure) was used as a raw material, phosphoric acid could be recovered efficiently and relatively safely by the production method for producing phosphoric acid in accordance with an embodiment of the present invention. According to Table 18, the phosphoric acid recovery efficiency is higher in the case of using the acid extract by hydrochloric acid or nitric acid than in the case of using the acid extract by sulfuric acid. The phosphoric acid recovery efficiency of the system using hydrochloric acid or nitric acid reached 3.2 times to 3.4 times that of the system using sulfuric acid.

This is also shown in Fig. 21. According to the elution curve of the ion chromatography illustrated in Fig. 21, in the system using hydrochloric acid or nitric acid, few anions other than phosphate ions were contained except for chloride ions (hydrochloric acid was used in the step 5 to dissolve the precipitate). Meanwhile, in the system using sulfuric acid, sulfate ions were detected in addition to phosphate ions.

Results of this Example indicate that phosphoric acid can be extracted from commercially available bone meal (bone tissue heated under pressure). Thus, it has been proved that the production method for producing phosphoric acid in accordance with an embodiment of the present invention is a sustainable technique which is of lower energy cost and is unlikely to cause environmental destruction, as compared with a method for recovering phosphoric acid from phosphate rock and sewage sludge dry matter.

### [Example 14: Production of protease treatment liquid from commercially available bone meal]

By the following procedure, commercially available steamed bone meal (Omiya Green Service, K.K.) was solubilized to produce a protease treatment liquid.
1. To 1 g of the steamed bone meal, 10 mL of sulfuric acid (1N), hydrochloric acid (1N), or nitric acid (1N) was added, and a resultant mixture was shaken at 25°C for 24 hours.
2. A resultant acid extract was divided into a supernatant (acid extract) and a precipitate (bone residue) using a centrifuge, and the supernatant and the precipitate were collected.
3. To the precipitate, 10 mL of a citrate buffer solution (pH: 3) of 0.1 mol/L or a tris hydrochloride buffer solution (pH: 8) of 0.1 mol/L was added.
4. In accordance with S5 of Fig. 1, a protease treatment liquid was prepared from the precipitate. As the protease, Newlase F3G (Amano Enzyme Inc.) or Papain W-40 (Amano Enzyme Inc.) was used, and treatment was carried out with a procedure identical with Example 1-5. Specifically, the following treatment was carried out.
   - Newlase F3G system: protease concentration = 1% (w/w) of initial bone weight; buffer solution = citrate buffer solution (pH: 3); temperature = 37°C; immersion time = 3 days
   - Papain W-40 system: protease concentration = 1% (w/w) of initial bone weight; buffer solution = tris hydrochloride buffer solution (pH: 8); temperature = 37°C; immersion time = 3 days
5. The reaction liquid was centrifuged to be divided into a precipitate and a supernatant.

### [Results]

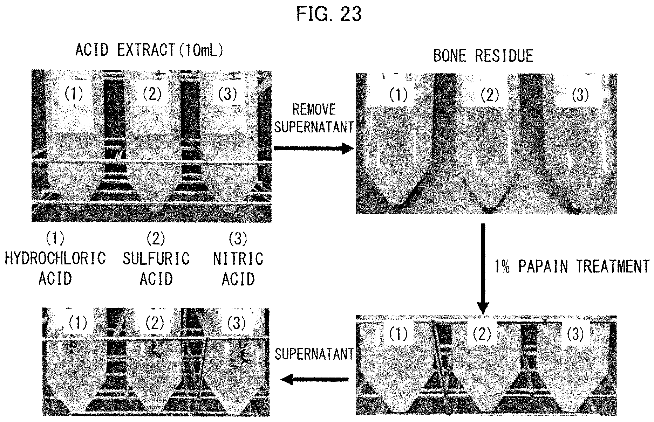

Results are shown in Figs. 22 and 23. The commercially available steamed bone meal is a mixture of a pig bone and a chicken bone which have been heated under high pressure. Even by using this bone meal as a raw material, a protease treatment liquid could be prepared by the production method for producing a fertilizer in accordance with an embodiment of the present invention. The protease treatment liquid as it is can be used as a fertilizer, or the protease treatment liquid can be used as a raw material for a bone solubilization liquid B. In a liquid fertilizer, a fast-acting plant growth effect is expected, which cannot be expected of solid bone meal. Moreover, the liquid fertilizer can be applied to hydroponic culture and foliar spray.

For the protease treatment using Newlase F3G, the bone residue after treatment with hydrochloric acid was almost entirely dissolved by the protease treatment. The bone residue after treatment with nitric acid was entirely dissolved by the protease treatment. For the bone residue after treatment with sulfuric acid, a white precipitate was found even after the protease treatment. This precipitate seems to be calcium sulfate, and at least a part of a protein component is considered to be degraded.

For the protease treatment using Papain W-40, the bone residue after treatment with hydrochloric acid or nitric acid was almost entirely dissolved by the protease treatment. For the bone residue after treatment with sulfuric acid, a white precipitate was found even after the protease treatment. This precipitate seems to be calcium sulfate, and at least a part of a protein component is considered to be degraded.

From the above results, it has been found that, in obtaining a protease treatment liquid while using an acid extract using sulfuric acid as a raw material, it is sometimes necessary to remove a precipitate by centrifugal separation or the like. In obtaining a protease treatment liquid using an acid extract using hydrochloric acid or nitric acid as a raw material, removal of a precipitate is not necessary. In this respect, it is preferable to use hydrochloric acid or nitric acid in the acid treatment step. Moreover, for the centrifuged supernatant of the solution after protease treatment, the supernatant derived from treatment with sulfuric acid was transparent, and the supernatant derived from treatment with nitric acid or hydrochloric acid was turbid. From the results, it has been found that, in order to more completely degrade the bone residue, it is more preferable to carry out acid treatment using hydrochloric acid or nitric acid. Note, however, that a protease treatment liquid can be prepared even by using sulfuric acid.

### [Example 15: Measurement of peptide concentration in acid extract obtained using commercially available bone meal as raw material]

By the following procedure, an acid extract was prepared from commercially available steamed bone meal (Omiya Green Service, K.K.). A peptide concentration contained in the acid extract was measured.
1. To 1 g of the steamed bone meal, 10 mL of sulfuric acid (1N), hydrochloric acid (1N), or nitric acid (1N) was added, and a resultant mixture was shaken at 25°C for 24 hours.
2. A resultant acid extract was divided into a supernatant and a precipitate using a centrifuge. The supernatant was collected as an acid extract.
3. To 0.5 mL of the supernatant, a potassium hydroxide aqueous solution of 0.4 mol/L was added to precipitate discrete calcium.
4. A peptide concentration was measured in the supernatant from which the precipitate was removed from the reaction liquid obtained in the step 3. In the measurement, a protein assay BCA kit (product number 297-73101, FUJIFILM Wako Pure Chemical Corporation) was used. The measurement was carried out in accordance with a product manual. An absorbance at 540 nm was measured. As the absorbance increases, the peptide concentration is higher.
5. A precipitate was collected from the reaction liquid obtained in the step 3. In that case, only a system treated with hydrochloric acid or nitric acid in the step 1 was collected.
6. The precipitate was completely dissolved in hydrochloric acid of 1N. Subsequently, 0.4 mol/L of potassium hydroxide was added again to precipitate calcium phosphate. This dissolution-precipitation process was repeated three times.
7. After the first and third repetitions were completed in the step 6, a peptide concentration in the supernatant from which the precipitate had been removed from the reaction liquid was measured. In the measurement, a protein assay BCA kit (product number 297-73101, FUJIFILM Wako Pure Chemical Corporation) was used. The measurement was carried out in accordance with a product manual. An absorbance at 540 nm was measured. As the absorbance increases, the peptide concentration is higher.

### [Table 19]

**Table 19**

| Peptide contained in supernatant after removal of Ca phosphate | | | |
|---|---|---|---|
| | Hydrochloric acid | Nitric acid | Sulfuric acid |
| Absorbance at 540 nm | 0.652 | 0.309 | 0.520 |

| Peptide remaining in phosphoric acid precipitate | | | | |
|---|---|---|---|---|
| | Hydrochloric acid | | Nitric acid | |
| | 1st repetition | 3rd repetition | 1st repetition | 3rd repetition |
| Absorbance at 540 nm | 0.319 | 0.013 | 0. 167 | 0.002 |

### [Results]

Results are shown in Table 19. A peptide was detected in the acid extract which was prepared using commercially available steamed bone meal as a raw material. The peptide concentration was higher, in descending order, in the acid extract derived from treatment with hydrochloric acid, the acid extract derived from treatment with sulfuric acid, and the acid extract derived from treatment with nitric acid. This result has shown that, in particular, the acid extract derived from treatment with hydrochloric acid contains a large amount of peptide. The peptide is an organic component contained in bone tissue. Therefore, it has been suggested that the acid extract contains a component which brings about a plant growth effect.

In Example 5, it was shown that the acid extract hardly contained a protein component which was stained with the silver staining kit (see also Fig. 14). In regard to the reason for this, in Example 5, it has been assumed that a peptide bond of a protein was cleaved to be lowmolecular peptide and amino acid. This Example provides support for this assumption.

A peptide is an impurity in producing phosphoric acid. It has been found that the peptide as an impurity can be almost removed by repeating the dissolution-precipitation process of calcium phosphate (step S13 and step S14 in Fig. 3). As shown in Table 19, the peptide concentration was decreased to 1/100 by repeating the dissolution-precipitation process three times. From this result, it has been suggested that, particularly from the acid extract derived from treatment with hydrochloric acid or nitric acid, the peptide can be successfully removed to heighten the phosphoric acid concentration.

### Industrial Applicability

The present invention can be used in growing a plant.

## Claims

1. A production method for producing a fertilizer containing phosphoric acid, said production method comprising at least one of:
a step 1 of producing a fertilizer from a bone solubilization liquid A which has been obtained by treating bone tissue with a solution containing both an acid and a protease;
a step 2 of producing a fertilizer from an acid extract which has been obtained by treating bone tissue with an acid; and
a step 3 of producing a fertilizer from a protease treatment liquid which has been obtained by treating, with a protease, bone tissue which has been treated with an acid.

2. The production method as set forth in claim 1, further comprising, in addition to the step 2 and the step 3:
a step 4 of producing a fertilizer from a bone solubilization liquid B which has been obtained by mixing the acid extract and the protease treatment liquid.

3. The production method as set forth in claim 1, wherein:
said production method comprises the step 1 or the step 2; and
bone tissue is treated with an acid which is at least one selected from the group consisting of nitric acid, hydrochloric acid, formic acid, and sulfuric acid.

4. The production method as set forth in claim 1, wherein:
said production method comprises the step 1 or the step 3; and
bone tissue is treated with a protease having an optimum pH of 1.5 to 8.0.

5. The production method as set forth in claim 1, wherein:
said production method comprises the step 1 or the step 2; and
bone tissue is treated with an acid at 5°C to 60°C.

6. The production method as set forth in claim 1, wherein:
said production method comprises the step 1 or the step 2; and
bone tissue is treated with an acid of 0.6 mol/L to 2.0 mol/L.

7. The production method as set forth in claim 1, wherein:
said production method comprises the step 1 or the step 2; and
bone tissue is treated with an acid for 6 hours to 48 hours.

8. The production method as set forth in claim 1, further comprising:
a pretreatment step of pretreating the bone tissue prior to the step 1 or the step 2,
the pretreatment being at least one selected from the group consisting of heating of the bone tissue, heating of the bone tissue under pressure, and irradiation of the bone tissue with microwaves.

9. The production method as set forth in claim 8, wherein:
in the pretreatment step, a protein contained in the bone tissue is denatured.

10. A fertilizer which is obtained by a production method recited in any one of claims 1 through 9.

11. The fertilizer as set forth in claim 10, wherein:
said fertilizer is a liquid fertilizer.

12. A fertilizer, comprising:
phosphoric acid; and
a peptide fragment which is derived from at least one selected from the group consisting of type I collagen, alpha-2-HS-glycoprotein, periostin, biglycan, and SPARC.

13. The fertilizer as set forth in claim 12, wherein:
in the peptide fragment, activity of a protein from which the peptide fragment is derived is lost.

14. The fertilizer as set forth in claim 12, wherein:
a molecular weight of the peptide fragment is 10,000 Da or less.

15. The fertilizer as set forth in claim 12, wherein:
a concentration of the phosphoric acid contained in the fertilizer is 280 mM or more.
